# EUROPEAN PATENT SPECIFICATION

(11) **EP 1 115 382 B1**
(45) Date of publication and mention of the grant of the patent: **19.05.2010**
(21) Application number: 99969329.4
(22) Date of filing: 22.09.1999
(51) Int. Cl.: A61K 9/127, A61K 39/00, C12N 15/62, C12N 15/85

(54) **IMMUNOGENIC LIPOSOME COMPOSITIONS**
IMMUNOGENE LIPOSOMZUSAMMENSETZUNGEN
COMPOSITIONS LIPOSOMIQUES IMMUNOGENIQUES

(30) Priority: 22.09.1998 US 101351 P; 21.09.1999 US 400723 P
(43) Date of publication of application: 18.07.2001
(73) Proprietor: Molecular Express Inc., Los ANgeles, CA 90061 (US)
(72) Inventor: FUJII, Gary, Torrance, CA 90504 (US); CRAMER, Donald, V., Agoura Hills, CA 91301 (US); ERNST, William, A., West Los Angeles, CA 90025 (US); ADLER-MOORE, Jill, Altadena, CA 91001 (US); PERRY, L., Jeanne, Los Angeles, CA 90046 (US)
(74) Representative: Smart, Peter John
(86) International application number: PCT/US1999/020880
(87) International publication number: WO 2000/016746

(56) References cited:
- EP-A- 0 247 497
- WO-A-87/02250
- FR-A- 2 754 543

## Description

### Background of the Invention

The use of vaccines historically has provided a safe and effective method of protecting large populations against a wide variety of infectious diseases. Immunization against viral and other microbial organisms is generally safe and effective, and has been responsible for much of the improvement in longevity experienced by individuals within developed nations. However, a number of adverse side effects can occur, depending upon the nature of the vaccine and the specific immunogen. Inadequate inactivation of intact virus has in the past led to unintended infection instead of protection following vaccination (Tigertt, 1959, Military Med. 124:342). Occasionally, immunization with intact organisms, such as influenza, can precipitate the development of autoimmune diseases directed against normal tissues such as Guillain Barre syndrome (Langmuir et al., 1984, J. Epidemiol. 119:841). In addition to the agents themselves, the presence of substances within the cells or complex media may allow for the induction of severe allergic reactions to foreign proteins (Yamane and Uemura, 1988, Epidem, Inf. 100:291). Since effective vaccination procedures frequently require multiple immunizations, these adverse events can reduce the effectiveness of the vaccine and may reduce public acceptance of the vaccination procedure. Modem molecular biologic techniques have been tested recently in an attempt to provide improved safety and efficacy of new vaccine preparations. Potential strategies currently under investigation include; vaccines based upon recombinant DNA techniques (Conry et al., 1994, Cancer Res. 54:1164; Hu et al., 1988, J. Virol. 62:176), generation of simple synthetic peptides as antigens (Nardelli et al., 1992, Vaccines 8:1405; Watari et al., 1987, J. Exp. Med. 165:459), and the direct injection of genetic material into tissues to stimulate protective responses (Ulmer et al., 1993, Science 259:1745). In particular, the use of simple peptide antigens to induce specific immunization responses has been the focus of many studies. This strategy is attractive because it has the potential to provide immunological specificity, tighter control of manufacturing processes, and elimination of most of the secondary sources of materials or contaminants associated with the production of the immunogen.

The use of purified proteins or peptide fragments for vaccination, however, depends upon the delivery of the material to the site of immunization by an effective antigen carrier. Potentially, one of the safest carriers has been lipid/liposome-based vaccine complexes. Liposomes have long been established as a commercially viable technology because they can reduce drug toxicities, prolong circulation in the bloodstream, and alter the biodistribution and pharmacokinetics of drug molecules (Fujii, 1996; Vesicles, ed. M. Rosoff, Marcel Dekker, New York NY, p. 491). When administered *in vivo*, liposomes circulate in the blood and are removed by the monocyte/macrophage phagocytic system, particularly in the liver, spleen, lymph nodes, and lung tissues (Claasen, 1996; Vesicles, ed. M. Rosoff, Marcel Dekker, New York NY, p.649). The ability of liposomes to direct the pattern of antigenic distribution suggests that a liposomal immunogen would be maximally exposed to the immune system. To date, several lipid based systems have been tested, including peptides conjugated to lipids (Nardelli et al., 1992, Vaccines 8:1405; Watari et al., 1987, J. Exp. Med. 165:459), reconstitution of whole protein subunits in the presence of lipids (Morein and Simons, 1985, Vaccines 4:166; Gregoriadis, 1995, Tibtech 13:527), and association of proteins with pre-formed liposomes (Therien and Shahum, 1989, Immunol. Lett. 22:253; Alving et al., 1985, Vaccines 4:166). While these strategies have been shown to be immunologically active, technical difficulties associated with the large-scale production of the proteins and their lipid carriers, as well as cost constraints, make them less attractive as a commercial technology. Thus, an improved system or approach for preparing protein antigens is needed to take advantage of the potential offered by liposomes in vaccine development.

EP-A-0247497 discloses functionalized liposomes containing a high-molecular-weight amphiphilic compound such as LPS. An antigen such as an antibody can be immobilized on the liposomes by using the amphiphilic compound as a spacer.

FR-A-2754543 discloses liposomes comprising *Bordetella* FHA, a heterologous protein and lipids.

### Summary of the Invention

The present invention provides an immunogenic liposome composition comprising vesicle-forming lipids and an antigenic construct which is an aqueous soluble fusion product comprising one or more antigenic determinants and a hydrophobic domain, wherein the the hydrophobic domain is a peptide and according to a preferred embodiment, consists of from about 15 to about 500 amino acid residues, more preferably less than about 300, and most preferably less than about 50 residues where at least one or more of the amino acids are selected from the group consisting of Ala, Asn, Cys, Gln, Gly, His, Ile, Leu, Met, Phe, Pro, Ser, Thr, Trp, Tyr and Val.

According to one embodiment of the present invention, the immunogenic liposome composition further comprises one or more adjuvants. Examples of suitable adjuvants include lipophilic molecules such as Lipid A and other lipopolysaccharides, Quil A, QS21, MF59, P₃CSS, MTP-PE, as well as water-soluble molecules, including cytokines such as IL-2, IL-4, IL-12, the interferons, and the like. Other examples of cytokines are provided in Table 1 below.

According to another embodiment of the invention, the antigenic construct further comprises a linker region linking the antigenic determinant(s) with the hydrophobic domain. In a preferred embodiment, the linker region is a peptide, consisting of from 1 to about 200 amino acid residues. Preferably, the amino acid residues are naturally occurring amino acids such as Ala, Arg, Asn, Asp, Cys, Glu, Gln, Gly, His, Ile, Leu, Lys, Met, Phe, Pro, Ser, Thr, Trp, Tyr and/or Val.

The present invention enables a method of inducing an immunogenic response in a host animal, comprising administering an immunogenically effective amount of a liposome composition described above. Although the host may be any animal including poultry, preferably, the host animal is a mammal, and more preferably, a human. In certain preferred embodiments, the immunogenic liposome composition further comprises one or more suitable adjuvants.

The present invention further enables a DNA cassette for insertion into a vector comprising sequences which encode an aqueous soluble immunogenic fusion protein, where the fusion protein comprises a hydrophobic protein domain and one or more antigenic determinants.

The present invention also provides a method of making an immunogenic liposome composition comprising: expressing a gene that encodes an aqueous soluble fusion protein comprising an antigenic determinant and a hydrophobic domain; dissolving lipids and the fusion protein in a suitable organic solvent or mixture of organic solvents; forming a lipid film by evaporating the organic solvent; hydrating the lipid film; and dispersing the hydrated lipid film to form an immunogenic liposome composition.

The present invention further provides a method of making an immunogenic liposome composition comprising: expressing a gene that encodes an aqueous soluble fusion protein comprising an antigenic determinant and a hydrophobic domain; dissolving the fusion protein in an aqueous buffer; hydrating either lipid powders or lipid films with the buffer containing the fusion protein; and dispersing the lipid powders or films to form an immunogenic liposome composition.

In another embodiment, the present invention provides a method of making an immunogenic liposome composition comprising: expressing a gene that encodes an aqueous soluble fusion protein comprising an antigenic determinant and a hydrophobic domain; dissolving the fusion protein in an aqueous buffer; and adding the fusion protein to pre-formed liposomes to form an immunogenic liposome composition.

In yet a further embodiment, the present invention provides a method of making an immunogenic liposome composition comprising: chemically synthesizing a fusion protein comprising an antigenic determinant and a hydrophobic domain; and preparing an immunogenic liposome composition by any of the methods described above.

As used herein, "associated with the membrane" means that the hydrophobic domain is at least partially embedded in the liposome membrane, and preferably is not covalently linked to the vesicle-forming lipids. The hydrophobic domain may also be bonded to a lipid fatty acid "tail" which itself is embedded in the membrane.

### Brief Description of the Drawings

Figure 1 shows the effectiveness of a liposome composition according to the present invention in protecting against HSV2.
Figure 2 shows a comparison of the effectiveness of a liposome composition according to the present invention with other vaccine compositions.
Figure 3 shows a survival curve of mice demonstrating the ability of liposomal HSV2g(1-23)-HD to elicit a protective immune response.

### Detailed Description of the Invention

The present invention provides an antigen delivery system designed to improve the effectiveness and safety of immunization against a variety of microbial agents and cancers. The immunogen can be encoded by a gene cassette that consists of a hydrophobic domain (HD) fused to a specific antigen sequence. A plasmid containing the nucleic acid sequences encoding the HD and the antigenic epitope is prepared and expressed in a suitable host, such as, for example, *E. coli, P. pastoris*, insect cells, COS-1 cells or CHO cells *(*Genetic Engineering News, 18:17 (1998*)).* Once the protein has been expressed and purified, it is formulated in a liposome. In the presence of a membrane, the protein associates with the lipid bilayer to form a stable structure with the hydrophobic portion associated with the membrane and the antigenic epitope oriented toward the aqueous medium. The plasmid can be engineered with restriction sites at key locations so that different antigenic epitopes can easily be substituted, thus providing the ability to utilize different antigenic epitopes to provide maximal protection following immunization. One of the unique features of this cassette is that the protein component can be produced in host cells in large quantities, readily purified, and then incorporated into liposomes. This provides maximal flexibility for determining the most appropriate epitope that promotes protection from microbial agents or from cancers, while maintaining the ultimate goal of large-scale preparation and commercial distribution of the vaccine.

Thus, the present invention provides several advantages: (1) epitopes can be readily changed to provide for maximal flexibility in vaccine design; (2) multiple epitopes can be inserted into the carrier molecule; (3) large antigen sequences (i.e., envelope proteins or receptor domains) or subunits can be included if desired and; (4) the expressed carrier protein is water soluble and can be easily purified using standard protein preparation methods. Synthesis of the antigenic construct as an aqueous soluble fusion product minimizes potential large-scale production problems caused by the hydrophobic region of the protein. Thus, construction of a protein possessing a hydrophobic domain for liposome membrane insertion and yet is still water soluble, would be a major advantage. In some circumstances, a minor amount of solubilizing agent, such as guanidine, urea, sodium dodecylsulfate, octyl glucoside or deoxycholate, may be used during the purification process. The present invention also provides constructs comprising two or more antigens. Such constructs can be represented as:

H₂N - Antigen site I - HD - Antigen site II - COOH

The invention also contemplates the use of naturally derived or synthetic single transmembrane helices, or helix bundles (2-12). The antigen sites may be located at the N- or C- terminus or situated between loops formed between the individual strands of the bundle.

The term "antigen" as used herein, refers to any substance (including a protein or protein-polysaccharide, lipopolysaccharide, microbial subunit, whole pathogen, or cancer markers) that, when foreign to the host animal, and upon gaining access to the tissue of such an animal, stimulates macrophages, antigen presenting cells and the formation of antigen specific antibodies, and reacts specifically *in vivo* or *in vitro* with such antibodies. Moreover, the antigen stimulates the proliferation and/or activation of T-lymphocytes with receptors for the antigen and cross-reacting antigens (e.g., natural killer (NK) cells, cytotoxic T cells and T helper cells), and can react with the lymphocytes to initiate the series of responses designated cell-mediated immunity. In the immunogenic compositions of the present invention it is preferred that the antigen be present in an amount of about 0.1 - 20 mg/ml antigen-HD. More preferably, the antigen is present in an amount of about 0.5 - 5 mg/ml antigen-HD.

An "antigenic determinant" is that portion of an antigen or antigenic construct that determines its immunological specificity. Commonly, an antigenic determinant, or hapten, is a peptide, protein or polysaccharide in naturally occurring antigens. In artificial antigens, it may be a low molecular weight substance such as an arsanilic acid derivative. An antigenic determinant will react specifically *in vivo* or *in vitro* with an antibody or T-lymphocytes induced by an antigenic form of the antigenic determinant (e.g., the antigenic determinant attached to an immunogenic substance).

Antigenic determinants, which may be used in practicing the present invention, may be derived from, by way of example only, antigens presented below:

**Viral pathogens**

| *Virus* | *Antigen* | *Reference* |
|---|---|---|
| Bluetongue | Structural | Murray and Eaton, 1996, Austral. Vet. J. 73: 207 |
| Bovine Herpes (IBR) | | Franz et al., 1996, Veterini Medicina 41: 213 |
| Bovine Virus Diarrhea | E2 | Bruschke et al., 1997, Vaccine 15: 1940 |
| | | Ludeman and Katz, 1994, Biologicals 22: 21 |
| Cytomegalovirus^{a} | gp58 | Wagner et al., 1992, J. Virol. 66: 5290 |
| | GB | Wang et al., 1996, J. Inf. Dis. 174: 387 |
| | Polyepitope Ag | Thomson et al., 1996, J. Immunol. 157: 822 |
| | PrM | Fonseca et al., 1994, Vaccine 12: 279 |
| | E | Fonseca et al., 1994, Vaccine 12: 279 |
| | E/NS1 | Venugopal and Gould, 1994, Vaccine 12: 967 |
| | NS1/NS2 | Green et al., 1997, Virol. 234: 383 |
| | Envelope B | Simmons et al., 1998, Am. J. Trop. Med. Hyg. 58: 655 |
| | type 4 capsid | Gagnon et al., 1996, J. Virol. 70: 141 |
| Distemper (Canine) | F/H | Pardo et al., 1997, Am. J. Vet. Res. 58: 833 |
| Ebola | GPMP | Vanderzanden et al., 1998, Virol. 246: 134 |
| Epstein-Barr | EBNA 1,2,3,4,6 | Moss et al., 1992, Sem. Immunol. 4: 97 |
| Foot and Mouth Disease | VP1 | Filgueria et al., 1995, Vaccine 13: 953 |
| Hepatitis A | A/B combo Ag | Bruguera et al., 1996, Vaccine 14: 1407 |
| Hepatitis B | HbsAg | Thanavala et al., 1995, PNAS 92: 3358 |
| | | Skelly et al., 1981, Nature 290: 51 |
| Hepatitis C | E2/NS1 | Weiner et al., 1992, PNAS 89: 3468 |
| | | Taniguchi et al., 1993, Virol. 195: 297 |
| | NS3 | Khudyakov et al., 1995, Virol. 206: 666 |
| | NS4 | Khudyakov et al., 1995, Virol. 206: 666 |
| | NS5 | Khudyakov et al., 1995, Virol. 206: 666 |
| | E1 | Lechner et al., 1998, Virol. 243: 313 |
| Herpes Simplex I | GD | Long et al., 1984, Inf. Immun. 37: 761 |
| Herpes Simplex II^{c} | GB | McDermott et al., 1989, Virol. 169: 244 |
| | GD | Long et al., 1984, Inf. Immun. 37: 761 |
| Herpes B | | Hunt and Melendez, 1969, Lab. Animal Care 19: 221 |
| Hog Cholera | E1 | van Rijn et al., 1994, J. Virol. 68: 3934 |
| Human Immunodeficiency^{d} | | HIV Molecular Immunology Database |
| Human Papilloma | L1 | Suzich et al., 1995, PNAS 92: 11553 |
| | | Stauss et al., 1992, PNAS 89: 7871 |
| | E6 | Stauss et al., 1992, PNAS 89: 7871 |
| | E7 | Stauss et al., 1992, PNAS 89: 7871 |
| Influenza | HA/N | Laver and Webster, 1976, Virol. 69: 511 |
| | NP | Martinon et al., 1993, Eur. J. Immunol. 23: 1719 |
| Japanese Encephalitis^{b} | E | Kimura-Kuroda and Yasui, 1988, J. Immunol. 141: 3606 |
| Marburg | GP | Hevey et al., 1997, Virol. 239: 206 |
| Marek's Disease (poultry) | GA | Boyle and Heine, 1993, Immun. Cell Biol. 71: 391 |
| | GB | Boyle and Heine, 1993, Immun. Cell Biol. 71: 391 |
| Measles | HA | Cordoso et al., 1998, J. Virol. 72: 2516 |
| | NP | Cordoso et al., 1998, J. Virol. 72: 2516 |
| Norwalk | 56kD capsid | Ball et al., 1998, J. Virol. 72: 1345 |
| Newcastle Disease (poultry) | | F Reddy et al., 1996, Vaccine 14: 469 |
| | HN | Reddy et al., 1996, Vaccine 14: 469 |
| Parainfluenza | F/HN | Morein et al., 1983, J. Gen. Virol. 64: 1557 |
| Parvovirus (Canine) | VP2 | Lopez de Turiso et al., 1992, J. Virol. 66: 2748 |
| Rabies | G | Wiktor et al., 1984, PNAS 81: 7194 |
| Respiratory Syncytial | F protein | Falsey and Walsh, 1996, Vaccine 14: 1214 |
| | FG subunit | Neuzil et al., 1997, Vaccine 15: 525 |
| | | Hemming et al., 1995, Clin. Microbiol. Rev. 8: 22 |
| Rinderpest | H/F | Naik et al., 1997, Vaccine 15: 603 |
| Rotavitus^{c} | VP4 | Zhou et al., 1994, J. Virol. 68: 3955 |
| | VP7 | Zhou et al., 1994 ,J. Virol. 68: 3955 |
| | VP6 | Palombo et al., 1994, J. Gen. Virol. 75: 2415 |
| Rubella | E1 | Trudel et al., 1985, J. Virol. Meth. 12: 243 |
| Varicella-Zoster | GE | Fowler et al., 1995, Virol. 214: 531 |
| | | Arvin, 1996, Inf. Clin. Dis. N. Amer. 10: 529 |
| Yellow Fever^{b} | E | Gould et al., 1986, J. Gen. Virol. 67: 591 |
| | NS1 (48kD) | Schleschinger et al., 1987, J. Immunol. 135: 2805 |

| | | |
|---|---|---|
| a. For a review, see Pass, 1996, Inf. Ag. Dis. 5: 240; Avery, 1998, Curr. Op. Cardiol. 13: 122. b. These viruses are all flaviviruses - reviewed in Venugopal and Gould, 1994, Vaccine 12: 966. c. For a review, see Adimora et al., 1994, Inf. Dis. Clin. N. Amer. 8: 859. d. For a listing of HIV antigens, see HIV Molecular Immunology Database, publ. Theoretical Biology and Biophysics, New Mexico (1995). e. For a listing of Rotavirus antigens, see "Rotavirus Antigens", Hoshino and Kapikian, 1994, Curr. Top. Microbiol. Immunol. 185:179. | | |

**Bacterial toxins***

| *Bacterium* | *Toxin* | *Reference* |
|---|---|---|
| Bacillus anthracis | Anthrax | Leppla, 1982, PNAS 79: 852 |
| Bordetella pertussis | Pertussis | Weiss and Hewlett, 1986, Ann. Rev. Microbiol. 40: |
| Clostridium botulinum | Botulinum | Simpson, 1981, Pharmacol. Rev. 33: 155 |
| Clostridium difficile | Difficile | Knoop et al., 1993, Clin. Microbiol. Rev. 6: 251 |
| Clostridium tetani | Tetanus | Eidels et al., 1983, Microbiol. Rev. 47: 596 |
| Corynebacterium diphtheriae | Diphtheria | Pappenheimer, 1977, Ann. Rev. Biochem. 46: 69 |
| Escherichia coli | Enterotoxin | Gill and Richardson, 1980, J. Inf. Dis. 141: 64 |
| Pseudomonas aeruginosa | Exotoxin A | Igiewski and Kabat, 1977, PNAS 72: 2284 |
| Shigella dysenteriae | Shiga | Keusch and Jacewicz, 1975, J. Inf. Dis. 131: S33 |
| Vibrio cholerae | Cholera | Finkelstein, 1973, Crit. Rev. Microbiol. 2: 553 |

| | | |
|---|---|---|
| * For a general review, see Middlebrook and Dorland, 1984, p.199. | | |

**Bacterial pathogens**

| *Bacterium* | *Antigen* | *Reference* |
|---|---|---|
| Bacillus sp | PA/LF/EF | Singh et al., 1998, Inf. Immun. 66: 3447 |
| Bordetella sp | Pertactin/P.69 | Anderson et al., 1996, Vaccine 14: 1384 |
| Borrelia sp | OspA/OspB | Ma et al., 1996, Vaccine 14: 1366 |
| | OspC | Mathiesen et al., 1998, Inf. Immun. 66: 4073 |
| Brucella sp | L7/L12 | Bachrach et al., 1994, Inf. Immun. 62: 5361 |
| | Omp16 | Tibor et al., 1994, Inf. Immun. 62: 3633 |
| Campylobacter sp | Omp 18 | Konkel et al., 1996, Inf. Imm |
| Chlamydia^{f} | MOMP | Sparling et al., 1994, PNAS 91: 2456 |
| Ehrlichia sp | | Rikihisa, 1991, Clin. Microbiol. Rev. 4: 286 |
| Escherichia sp | PAL | Chen and Henning, 1987, Eur. J. Biochem. 163: 73 |
| Haemophilus^{f} sp | P1 | Loeb, 1987, Inf. Immun. 55: 2612 |
| | P2 | Munson et al, 1983, J. Clin. Invest. 72: 677 |
| | P4 | Green et al., 1991, Inf. Immun. 59: 3191 |
| | P5 | Munson and Granoff 1985, Inf. Immun. 49: 544 |
| | P6 | Green et al., 1990, Inf. Immun. 58: 3272 |
| | Protein D | Akkoyunlu et al., 1996, Inf. Immu |
| | D15 | Thomas et al., 1990, Inf. Immun. 58: 1909 |
| | | Yang et al., 1998, Inf. Immun. 66: 3349 |
| | 98 kD protein | Kimura et al., 1985, Inf. Immun. 47: 253 |
| | HtrA | Loosmore et al., 1998, Inf. Immun. 66: 899 |
| Helicobacter pylori | Urease A | Michetti et al., 1994, Gastroenterol. 107: 1002 |
| | Urease B | Michetti et al., 1994, Gastroenterol. 107: 1002 |
| | Vac A | Kleanthous et al., 1998, Brit. Med. Bull. 54: 229 |
| | Catalase | Radcliff et al., 1997, Inf. Immun. 65: 4668 |
| Legionella sp | Pp1A | Ludwig et al., 1996, Inf. Immun. 64: 1659 |
| Leptospira sp | OMA | Brown et al., 1991, Inf. Immun. 59: 1772 |
| Listeria sp | LLO | Harty and Bevan, 1992, J. Exp. Med. 175: 1531 |
| Mycobacterium leprae | 35kD protein | Triccas et al., 1996, Inf. Immun. 64: 5171 |
| | 18kD | Baumgart et al., 1996, Inf. Immun. 64: 2274 |
| | Ag85A | Launois et al., 1994, Inf. Immun. 62: 3679 |
| Mycobacterium tuberculosum | MPB59 | Roche et al., 1994, Inf. Immun. 62: 5319 |
| | Ag85A | Launois et al., 1994, Inf. Immun. 62: 3679 |
| Mycoplasma | 90 kD | Franzoso et al., 1993, Inf. Immun. 61: 1523 |
| Neisseria^{f} sp | OMV | Anderson et al., 1997, Vaccine 15: 1225 |
| | Por | Sparling et al., 1994, PNAS 91: 2456 |
| Pseudomonas sp | OprF | Hughes et al., 1992, Inf. Immun. 60: 3497 |
| | OprI | Specht et al., 1996, Vaccine 14: 1111 |
| | O-polysaccharide | Hatano and Pier, 1998, Inf. Immun. 66: 3719 |
| Salmonella sp | OMP/Por | Alurkar and Kanat, 1997, Inf. Immun. 65: 2382 |
| | Vi | Plotkin and Bouveret-LeCam, 1995, Arch. Int. Med. |
| Staphylococcus sp | CP | Fatton et al., 1996, Inf. Immun. 64: 1659 |
| | RAP (38kD) | Balaban et al., 1998, Science 280: 438 |
| Streptococcus sp | M | Beachey et al., 1988, Vaccine 6: 192 |
| Treponema palladium^{f} | TROMP | Sparling et al., 1994, PNAS 91: 2456 |
| Yersinia sp | F1/V | Leary et al., 1997, Microbial Pathogen. 23: 167 |

| | | |
|---|---|---|
| f. For a review, see Adimora et al., 1994, Inf. Dis. Clin. N. Amer. 8: 859. | | |

**Fungal pathogens**

| *Organism* | *Antigen* | *Reference* |
|---|---|---|
| Aspergillus | | Kurup and Kumar, 1991, Clin. Microbiol. Rev. 4: 439 |
| Candida | Pral | Setandreu et al., 1998, J. Bacteriol. 180: 282 |
| Coccidioides | 48kD protein | Kirkland et al., 1998, Inf. Immun. 66: 424 |
| | CF | Yang et al., 1997, Inf. Immun. 65: 4068 |
| | PRA | Kirkland et al., 1998, Inf. Immun. 66: 3519 |
| Cryptococcus | | Mitchell and Perfect, 1995, Clin. Microbiol. Rev. 8: 515 |
| Histoplasma | | Deepe, 1997, Clin. Microbiol. Rev. 10: 585 |
| Paracoccidioides brasiliensis | | Brummer et al., 1993, Clin. Microbiol. Rev. 6: 89 |
| Pneumocystis carinii | GpA | Gigliotti et al., 1998, Inf. Immun. 66: 3179 |

**Parasitic pathogens**

| *Organism* | *Antigen* | *Reference* |
|---|---|---|
| Cryptosporidium | | Current and Garcia, 1991, Clin. Microbiol. Rev. 4: 325 |
| Entamoeba histolytica | SREHP (K2) | Stanley et al., 1990, PNAS 87: 4976 |
| | Ariel | Mai and Samuelson, 1998, Inf. Immun. 66: 353 |
| | 170kD | Lotter et al., 1997, J. Exp. Med. 185: 1793 |
| Giardia lamblia | VSP | Stager et al., 1997, Int. J. Parasitol. 27: 965 |
| Leishmania sp | gp63 | Kahl et al., 1990, Inf. Immun. 58: 3233 |
| | Nramp-1 | 1998, Inf. Immun. 66: 1910 |
| | TSA | Webb et al., 1998, Inf. Immun. 66: 3279 |
| Plasmodium sp | 27 kD protein | Contreras et al., 1998, Inf. Immun. 66: 3579 |
| | AMA-1 | Anderson et al., 1998, Vaccine 16: 240 |
| | CS | Nardin et al., 1998, Vaccine 16: 590; |
| | prot epitopes | 1998, Inf. Immun. 66: 2895 |
| | 195 kD protein | Patarroyo et al., 1987, Nature 328: 629 |
| | 55 kD protein | Patarroyo et al., 1987, Nature 328: 629 |
| | 35 kD protein | Patarroyo et al., 1987, Nature 328: 629 |
| Schistosoma sp | Sm28GST | Batione et al., 1987, Nature 326: 149 |
| Trypanosoma sp | 56 kD protein | Harth et al., 1994. Mol. Microbiol. 11: 261 |

**Cancers****

| *Neoplasm* | *Antigen* | *Reference* |
|---|---|---|
| Breast | MUC-1 | Denton et al., 1993, Canc. Lett. 70: 143 |
| Colon | CEA | Conry et al., 1994, Canc. Res. 54: 1164 |
| Leukemia | BCR/ABL | Chen et al., 1992, PNAS 89:1468. |
| Lymphoma | Self Ag | Kwak et al., 1992, New Eng. J. Med. 327: 1209 |
| Melanoma | MART-1 | Kawakami et al., 1994, PNAS 91: 3515 |
| | MAGE-1 | Traversari et al., 1992, J. Exp. Med. 176: 1453 |
| | p97 | Hu et al., 1988, J. Virol. 62: 176 |
| Ovarian | MUC-1 | Jerome et al., 1993, J. Immunol. 151: 1654 |
| Pancreatic | MUC-1 | Bashford et al., 1993, Int. J. Canc. 54: 778 |

| | | |
|---|---|---|
| ** For a general review, see Finn, 1993, Curr. Op. Immunol. 5: 701. | | |

Additional examples of antigens and pathogens which may be used are found in Milich, 1989 (Adv. Immunol. 45:195), Hoshino and Kaplan, 1994 (Curr. Top. Microbiol. Immunol. 185:179) or Venugopal and Gould, 1994 (Vaccine 12:966). According to one embodiment, the gp120 subunit of the env gene and p27 of the *gag* gene from Human Immunodeficiency Virus (HIV) are used. In another embodiment, the antigenic determinant may be derived from an antigen selected from HSV gD or gB, HIV gp120, CMV gB, HCV E2, INFV HA or NA, H. influenzae P5 or P6, fimbrial protein and Protein D.

As used herein, the term "hydrophobic domain" (HD) means any protein, peptide, lipid or molecule with a hydrophobic region or structure having a surface area greater than 5 nm². Examples of potential HDs include any transmembrane (TM) segment (e.g., glycophorin A; Tomita and Marchesi, 1975, Proc. Natl. Acad. Sci. USA 72:2964), the hydrophobic domain of cytochrome *b₅* (Taylor and Roseman, 1995, BBA 1278:35), the T domain of diphtheria toxin (Choe et al., 1992, Nature 357:216), domain II of Pseudomonas Exotoxin A (Allured et al., 1986, PNAS 83:1320), the 4-helix bundle of the penicillin sensory transducer (Hardt et al., 1997, Mol. Microbiol. 23:935), the 7-helix bundle of bacteriorhodopsin (Henderson and Unwin, 1975, Nature 257: 28), the 12-helix bundle of lac permease (Kaback et al., 1997, Curr. Op. Struct. Biol. 7:537) and the pore forming domain of colicin (Parker et al., 1989, Nature 357:93).

The term "vector" as used herein refers to a nucleic acid, (e.g., DNA) derived from a plasmid, cosmid, phagmid or bacteriophage or synthetically derived, into which fragments of nucleic acid may be inserted or cloned. The vector can contain one or more unique restriction sites for this purpose, and may be capable of autonomous replication in a defined host or organism such that the cloned sequence is reproduced. The vector molecule can confer some well-defined phenotype on the host organism which is either selectable or readily detected. Some components of a vector may be a DNA molecule incorporating regulatory elements for transcription, translation, RNA stability and replication, and other DNA sequences.

The term "DNA cassette" as used herein refers to genetic sequences within the vector which can express the fusion protein described herein. The nucleic acid cassette is positionally and sequentially oriented within the vector such that the nucleic acid in the cassette can be transcribed into RNA, and when necessary, translated into the fusion protein product. Preferably, the cassette has its 3' and 5' ends adapted for ready insertion into a vector, e.g., it has restriction endonuclease sites at each end.

While it is preferred that the antigenic constructs be produced using recombinant techniques, it is understood that the constructs can be synthesized by any means known in the art, such as, for example, chemical means. This may be particularly useful where the hydrophobic domain comprises a non-naturally occurring amino acid residue or mimetic. Thus, while the preferred embodiment makes use of recombinant methods to produce the antigenic fusion protein, the present invention also provides a method comprising: preparing the antigenic constructs by chemical synthetic means or by a combination of recombinant and chemical means and preparing an immunogenic lipid formulation as described above.

According to the present invention, the liposome plays a critical role in antigen delivery as the antigen-liposome complex is directly presented to the immune system following removal from the circulation by cells of the immune system. In addition, the choice of the immunostimulatory pathways can be altered by making changes to the lipid composition of the liposome. For example, different immunostimulatory molecules, such as Lipid A (Asano and Kleinerman, 1993, J. Immunother. 14:286), P₃CSS (Lex et al., 1986, J. Immunol. 137:2676), muramyl di- and tripeptide-PE (Fidler et al., 1981, PNAS 78:1680; Alving et al., 1985, Vaccines 4:166), and cationic lipids (Walker et al., 1992, PNAS 89:7915) can be formulated into the liposome to stimulate different immunological pathways. Other adjuvants such as, for example, MF59, Quil A, QS21 or alum, may be used in conjunction with the antigen-liposome complex. In addition, immunoregulatory molecules such as the cytokines can be added to elicit an immune response.

The liposomes used in the practice of the present invention can be prepared from a wide variety of vesicle-forming lipids including phosphatidyl ethers and esters, such as phosphatidylethanolamine (PE), phosphatidylserine (PS), phosphatidylglycerol (PG) and phosphatidylcholine (PC); glycerides, such as dioleoylglycerosuccinate; cerebrosides; gangliosides; sphingomyelin; steroids, such as cholesterol; and other lipids, as well as other excipients such as Vitamin E or Vitamin C palmitate (See also U.S. Pat. Nos. 4,235,871; 4,762,720; 4,737,323; 4,789,633; 4,861,597; and 4,873,089). Examples of PC-based lipids include, but are not limited to, (C-18) distearoylphosphatidylcholine (DSPC); (C-16) dipalmitoylphosphatidylcholine (DPPC); (C-14) dimyristoylphosphatidylcholine (DMPC); and (C-18, unsaturated) dioleylphosphatidylcholine (DOPC). It is preferred that the lipids be in a fluid phase at body temperature (approximately 38-39°C). Therefore, lipids with a Tₘ above body temperature, such as DSPC and DPPC, are less preferred (but may nevertheless still be useful). Lipids having a Tₘ below body temperature, such as DMPC or DOPC, are more preferred. In addition, it is preferred that the lipid formulation contain no more than about 10% cholesterol. Particularly preferred lipid compositions comprise about 0-10% cholesterol, about 0-15% PG and about 0-100% PC. In certain preferred embodiments, the composition may additionally comprise about 1-10% adjuvant(s); preferably, an adjuvant is present in an amount less than about 5%.

The preparation of liposomes is well known in the prior art. In general, liposomes have been made by a number of different techniques including ethanol injection (Batzri et al., 1973, Biochem. Biophys. Acta. 298:1015); ether infusion (Deamer et al., 1976, Biochem. Biophys. Acta. 443:629; Schieren et al., 1978, Biochem. Biophys. Acta. 542:137); detergent removal (Razin, 1972, Biochem. Biophys. Acta. 265:241); solvent evaporation (Matsumato et al., 1977, J. Colloid Interface Sci. 62:149); evaporation of organic solvents from chloroform in water emulsions (REV's) (Szoka Jr. et al., 1978, Proc. Natl. Acad. Sci. USA, 75:4194); extrusions of MLV's or EUV's through a nucleopore polycarbonate membrane (Olson et al., 1979, Biochem. Biophys. Acta. 557:9); freezing and thawing of phosopholipid mixtures (Pick, 1981, Archives of Biochem. and Biophysics, 212:186), as well as sonication and homogenization.

By convention, liposomes are categorized by size, and a 3-letter acronym is used to designate the type of liposome being discussed. Multilamellar vesicles are generally designated "MLV." Small unilamellar vesicles are designated "SUV," and large unilamellar vesicles are designated "LUV." These designations are sometimes followed by the chemical composition of the liposome. For a discussion of nomenclature and a summary of known types of liposomes, see Storm et al., 1998, PSIT, 1:19-31.

The present invention contemplates use of the liposome compositions with a suitable adjuvant. However, the present invention also contemplates use of the antigenic construct, which is not associated with a liposome, in combination with a suitable adjuvant. Thus, the vaccine or immunogenic composition is comprised of two components: an antigen linked to an hydrophobic domain; and a suitable adjuvant system. Examples of potential pharmaceutically acceptable carrier systems that may be used include, but are not limited to, liposomes, emulsions, Freund's adjuvant (complete or incomplete), alum, ISCOMS and enveloped viruses.

The methods of using the present invention will employ an immunogenically effective amount of the liposome composition, i.e., an amount of the composition that, in combination with any added excipients or carriers, will result in a detectable immunogenic response in the host. Where the composition will be used as a vaccine composition, the effective amount will be that amount which, in combination with any added excipients or carriers, will cause the host to produce a specific and sufficient immunological response so as to impart protection to the host from the subsequent exposure to a microbe (prophylactic vaccination), or to impart protection to the already diseased host (therapeutic vaccination).

The invention now being generally described, the same will be better understood by reference to the following detailed examples which are provided by way of illustration and are not intended to be limiting of the invention unless so specified.

### Examples

### Example I - HSV2

*Preparation of HSV2gD(1-23)-TM*. The N-terminal 23-amino acid segment from the gD envelope protein of HSV2 was coupled to a transmembrane (TM) segment by chemical synthesis on an automated peptide synthesizer. The synthesized peptide was cleaved by standard HF cleavage methods and purified by preparative HPLC using a Waters C18 column and 100% acetonitrile mobile phase with 0.1% TFA. The peptide was demonstrated to be at least 95% pure by mass spectrometry and capillary electrophoresis.

*Liposome preparation.* The liposomes were prepared by probe sonication according to previously described procedures (Fujii et al., 1997, Biochemistry 36:4959). Briefly, the lipids (with or without protein) are dissolved in an organic solvent such as chloroform/methanol. Thin lipid films are created by pipetting aliquots of the lipid solutions into round bottom glass tubes, and evaporating the solvent at 65°C under a stream of nitrogen gas. The films are placed under vacuum for at least eight hours to remove residual organic solvent. Preparation of the liposomes is accomplished by hydrating the lipid films with buffer and incubating the suspension at 65°C for 5-10 minutes before probe sonication. The liposomes are then filtered through a 0.22 µm filter to sterilize the preparation. The liposomes are sized by dynamic light scattering and the formation of aggregates followed for at least four weeks.

A particularly preferred liposomal HSV2gD(1-23)-TM formulation has a molar ratio of 15:2:3 DMPC:Chol:DNWG (dimyristoylphosphatidylcholine:cholesterol:-dimyristoyl-phosphatidylglycerol), with 2.5% by weight Lipid A.

### Vaccination procedures and testing for induction of immune response to viral

*antigen*. BALB/c or C57BL/6 female mice (6-8 weeks old) are subcutaneously injected once per week for two, three, or four weeks with the test vaccine preparation or buffer. The sites of injection are monitored for adverse reactions such as the development of granulomas and/or scab formation. When the animals are immunized with the vaccine via the intranasal route, they are anesthetized with halothane, and the composition (10-20 µl) administered to the nares for inspiration using a sterile tip on a micropipettor (Gallichan et al., 1993, J. Inf. Dis. 168:622).

At regular intervals during the vaccination procedure, and after viral challenge, the immune response of the mice to the viral antigen is measured. The viral neutralization antibody test is performed using 24-well cluster plates containing 1x10⁵ BHK cells to which have been added different dilutions of mouse serum mixed with virus. Following 48-72 hours of incubation at 37°C in a CO₂ incubator, the cell monolayers are examined for cytotoxicity, and the titer of neutralizing antibody assayed. Precipitating antibodies to the virus are measured by incubating different dilutions of serum with 5-10 µg of viral antigen or 25-50 µl of HSV2 stock (1x10⁵ PFU). Liposomes containing the antigenic epitope are also used as targets for an antibody/ELISA assay as described earlier (Tuso et al., 1993, Transplantation 55:1375). The results of the latter assay will be used to measure the level of binding and isotype configuration of the antibodies.

A delayed type hypersensitivity response to the viral antigen in vaccinated mice is tested using a footpad assay. Vaccinated animals are anesthetized with halothane and injected with 50 µl of ultraviolet-inactivated HSV2 in one hind footpad and 50 µl lysed uninfected cells in the other hind footpad. Twenty-four, forty-eight and seventy-two hours later, the degree of footpad swelling of the mice is measured using a Vernier caliper and compared to the baseline measurements. The T cell activity of these animals is also measured by the production of cytokines in response to incubation of splenic T cells with viral antigen. The spleens are removed from the vaccinated animals and spleen homogenates prepared by gently breaking up the spleens with a sterile plunger and pushing the cells through a nylon screen mesh. The cell suspensions are rinsed and plated at 1x10⁶ cells/well in a 96-well microtiter plate. Following incubation of the cells at 37°C for 3 to 4 days with different amounts of viral antigen, the supernatants from the wells are harvested and tested for the presence of cytokines. Commercially available cytokine ELISA kits are used to define the cytokine profile (IL-2, IL-4, IL-6, IL-10, IL-12, IFN-γ, β-actin, and TNF-α from PharMingen, Inc. (San Diego, CA)). Detection of the cytokine protein in tissue culture supernatants by sandwich ELISA is performed by using monoclonal antibodies (mAb) specific for the particular cytokines. Briefly, ELISA plates are coated overnight with a rat anti-mouse cytokine mAb. Plates are blocked with 3% fetal calf serum in PBS for 2 hours, then aliquots of each test sample are added to each well. Cytokine specific biotinylated rat anti-mouse mAb is added to each well followed by avidin peroxidase. A color reaction is achieved by the addition of colorimetric substrates. Plates are read in an ELISA spectrophotometer and cytokine concentrations calculated from a standard curve obtained from control recombinant cytokines.

### HSV2 mouse encephalitis model for testing liposomal vaccine preparations.

Vaccinated or unvaccinated (control) BALB/c female mice (10-12 weeks old) are intraperitoneally challenged with a lethal dose (1x10⁵ PFU) of brain passaged HSV2. Those mice challenged intravaginally with a lethal dose of HSV2 are first pretreated with subcutaneous estrogen one week prior to challenge and day (-1) prior to challenge. They are then anesthetized with an intraperitoneal injection of acepromazine and ketamine. The HSV2 (10-30 µl) is then administered intravaginally using a sterile tip on a micropipettor after first swabbing the vagina with a dry Calgiswab (McDermott et al., 1984, J. Virol. 51:747). Animals are monitored for 80 days after challenge for survival (daily), weight loss (every other day for the first two weeks and then once per week), appearance of fur, and neurological symptoms (decreased activity level, dragging or paralysis of limbs).

*Antigen-induced T cell proliferation*. Sensitization of the host to the gD antigen is measured using an antigen-induced T cell proliferation assay. Spleen homogenates are prepared by gently breaking up the spleens with a sterile plunger and pushing the cells through a nylon screen mesh. The cells are suspended in RPMI 1640 medium containing 10% fetal calf serum (FCS), 10 mM Hepes buffer, L-glutamine (2 mM), penicillin (25 IU/ml), and streptomycin (25 µg/ml). The cells are cultured at a concentration of 1 x 10⁶ cells/ml with or without gD (5-20 µg/ml) in 96-well U-bottom plates for 4 days in 5% CO₂. The cultures are pulsed with 20µl of resazurin dye for 3 hours and then the fluorescence is measured on a Cytofluor II (Perspective Biosystems, Inc., Farmington, MA).

*Cytokine-specific mRNA Analysis.* T cells from immunized and non-immunized animals are examined for cytokine-specific mRNA levels for the major cytokines that may reflect sensitization associated with immunization, including IL-2, IFN-γ, IL-4, IL-5, IL-6, and IL-10. The T cells are obtained from the spleens as described previously. Total RNA is isolated using a modification (Cosenza et al., 1995, Liver Transpl. Surg. 1:16) of a method described by Chomczynsky and Sacchi (Chomczynsky et al., 1987, Analyt. Biochem. 162:156). Fresh or frozen cells (2 x 10⁶) are disrupted in 1 mL of denaturing solution (4 mM guanidinium thiocyanate, 25 mM sodium citrate (pH 7.0), 0.5% sarcosyl, and 0.1 mM 2-mercaptoethanol). The RNA is precipitated by incubating with one volume of isopropanol overnight at -20°C. The concentration of total RNA is adjusted to 260 nM and the samples stored at -80°C until analyzed.

Cytokine analysis is conducted using modification of a method described by Cosenza et al. (Cosenza et al, 1995, Liver Transpl. Surg. 1:16). Single strand cDNA is prepared by transcription of 2 µg of total RNA in 20 µl of total reaction mixture using avian myeloblastosis virus reverse transcriptase (Boehringer Mannheim, Indianapolis, IN). Sequence specific oligonucleotide primers for murine cytokines (Table 1) are used to amplify DNA fragments of predetermined size for each of the cytokine genes of interest.

**Table 1. Oligonucleotide primers used for the semiquantive analyis of cytokines from mice***

| | Oligonucleotide | Nucleic Acid Sequence | Expected Target |
|---|---|---|---|
| II-2 | 5' | TGATGGGACCTACAGGAGCTCCTGAG (SEQ ID NO. 1) | 167 bp |
| | 3' | GAGTCAAATCCAGAACATGCCGCAG (SEQ ID NO. 2) | |
| II-4 | 5' | CGAAGAACACCACAGAGAGTGAGCT (SEQ ID NO. 3) | 180 bp |
| | 3' | GACTCATTCATGGTGCAGCTTATCG (SEQ ID NO. 4) | |
| II-5 | 5' | ATGACTGTGCCTCTGTGCCTGGAGC (SEQ ID NO. 5) | 242 bp |
| | 3' | CTGTTTTTCCTGGAGTAAACTGGGG (SEQ ID NO. 6) | |
| II-6 | 5' | TGGAGTCACAGAAGGAGTGGCTAAG (SEQ ID NO. 7) | 154 bp |
| | 3' | TCTGACCACAGTGAGGAATGTCCAC (SEQ ID NO. 8) | |
| II-10 | 5' | TCAAACAAAGGACCAGCTGGACAACATACTGC (SEQ ID NO. 9) | 426 bp |
| | 3' | CTGTCTAGGTCCTGGAGTCCAGCAGACTCAA (SEQ ID NO. 10) | |
| II-12 | 5' | TCGCAGCAAAGCAAGATGTG (SEQ ID NO. 11) | 315 bp |
| | 3' | GAGCAGCAGATGTGAGTGGC (SEQ ID NO. 12) | |
| IFN | 5' | AGCGGCTGACTGAACTCAGATTGTAG (SEQ ID NO. 13) | 843 bp |
| | 3' | GTCACAGTTTTCAGCTGTATAGGG (SEQ ID NO. 14) | |
| TNF-α | 5' | GGCAGGTCTACTTTGGAGTCATTGC (SEQ ID NO. 15) | 307 bp |
| | 3' | ACATTCGAGGCTCCAGTGAATTCGG (SEQ ID NO. 16) | |
| β-actin | 5' | TGGAATCCTGTGGCATCCATGAAAC (SEQ ID NO. 17) | 348 bp |
| | 3' | TAAAACGCAGCTCAGTAACAGTCCG (SEQ ID NO. 18) | |

The PCR mixture consists of 1 µl of cDNA, 2.5 µl of PCR 10x buffer, 1 µl of each 5' and 3' primers, 2.5 µl of 10x dNTPs (2 mmol/L) and 0.125 µl of *T. aquaticus* thermostable DNA polymerase (Boehringer Mannheim) in a final volume of 25 µl. Specific β-actin primers are used to amplify a 548 bp fragment as an internal control. The PCR mixture is covered with mineral oil and amplification conducted following incubation of the mixture for 7 minutes at 94°C, 38 cycles with 30 second denaturation at 94°C, 45 second primer annealing at 60°C, 60 second extension at 72°C, and a 7 minute incubation at 72°C. The PCR products are separated on agarose gel in the presence of ethidium bromide. Bands are visualized under UV light, photographed, and densitometrically quantified from the photograph using the Molecular Analyst software package (Bio-Rad, Richmond CA).

*Cytokine measurement by ELISA.* Commercially available cytokine ELISA kits are used to define the cytokine profile secreted by splenic CD4⁺ T cells isolated from immunized and non-immunized animals. Detection of the cytokine protein in tissue culture supernatants from splenic T cells by sandwich ELISA is performed by using monoclonal antibodies (mAb) specific for the particular cytokines. Briefly, ELISA plates are coated overnight with a rat anti-mouse cytokine mAb. Plates are blocked with 3% FCS in PBS for 2 hours, then aliquots of each sample are added to each well. Cytokine specific biotinylated rat anti-mouse mAb is added to each well followed by avidin peroxidase. A color reaction is achieved by the addition of colorimetric substrates. Plates are read in an ELISA spectrophotometer and cytokine concentrations calculated from a standard curve obtained from control recombinant cytokines.

*Anti-HSV2 CTL responses.* After vaccination, the presence of antigen-specific cytotoxic T lymphocytes (CTL) in splenic T cells are assessed. Splenic lymphocytes are obtained as described previously. Lymphocytes from each treatment group are pooled (n=5) and then cultured for 3 days without antigen at 10⁷ cells/well in 12-well culture plates. EL-4 (H2*^{b}*) target cells (ATTC) are incubated with a peptide corresponding to the HSV2 gB CTL epitope located between residues 495-505 (Hanke et al., 1991, J. Virol. 65: 1177) for H2*^{b}* restricted cells and incubated for 4 hours at 37 °C. Targets are washed, and 100µl titers containing 1 x 10⁴ cells are added to each well. Effector lymphocytes are washed, added to wells at various concentrations and cultured for 4 hours at 37 °C. Using the CytoTox 96 kit (Promega, Madison WI), the percent specific lysis is calculated from supernatant lactate dehydrogenase (LDH) measured in a standard ELISA plate reader (HTS 7000+ BioAssay Reader, Perkin Elmer, San Jose, CA) by recording the absorbance at 490 nm. The determination of HSV2-antigen specific lysis is made according to standard criteria. Data are expressed as percent specific lysis = 100 x [(experimental -effector spontaneous - target spontaneous)/(target maximum - target spontaneous)].

### Results

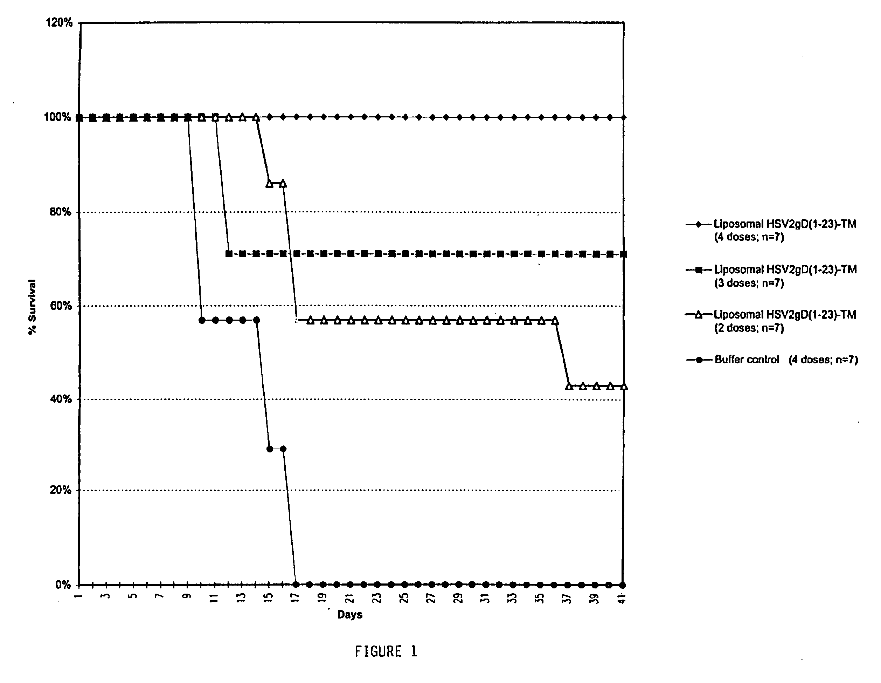

The survival curve presented in Figure 1 demonstrates the effectiveness of the liposomal-peptide in protecting the mice against systemic challenge with a lethal dose of HSV2 as well as establishing the number of inoculations required to achieve 100% protection. Figure 1 shows the number of doses of liposomal HSV2gD(1-23)-TM vaccine required to protect BALB/c mice from a lethal challenge with HSV2. Groups of 7 mice were given 2, 3, or 4 immunizations prior to challenge with a lethal dose of HSV2. With four vaccinations, none of the animals immunized with the liposomal vaccine exhibited neurological complications associated with infection by HSV2, nor were there any other signs of infection detected throughout the study. Most importantly, all of the mice survived the lethal challenge with HSV2. With two or three weekly immunizations, less than 100% of the mice were protected. In contrast, mice given four vaccinations of a placebo (i.e., buffer) were not protected from the HSV2 challenge.

Liposomal HSV2gD(1-23)-TM was also tested in three groups of C57BL/6 mice and found to elicit similar protective effects comparable to the results obtained with the BALB/c mice. Table 2 summarizes these results. Group 1 mice were vaccinated at weeks 1 and 4 subcutaneously; group 2 mice were vaccinated at week I subcutaneously and at week 4 intrarectally; and group 3 mice (control group) received no vaccinations. One week after the last vaccination (at week 4), the mice were challenged intraperitoneally with a lethal dose of HSV2, and were monitored for 3 weeks for morbidity and mortality. As with the BALB/C mice, the groups of mice receiving vaccinations with liposomal HSV2gD(1-23)-TM had significant numbers of survivors when vaccinated either by subcutaneous injection or when given a subcutaneous priming dose followed by mucosal boosters

**Table 2. Summary of results comparing the survival of C57BL/6 mice for various vaccination treatments with liposomal HSV2gD(1-23)-TM.**

| Group | Vaccination Treatment | Survival (n=7) |
|---|---|---|
| I | Week 1 - subcutaneous | 6/7 (86%) |
| | Week 4 - subcutaneous | |
| | | |
| 2 | Week 1 - subcutaneous | 5/7 (71%) |
| | Week 4 - intrarectal | |
| | | |
| 3 | Control (no vaccination) | 1/7 (14%) |

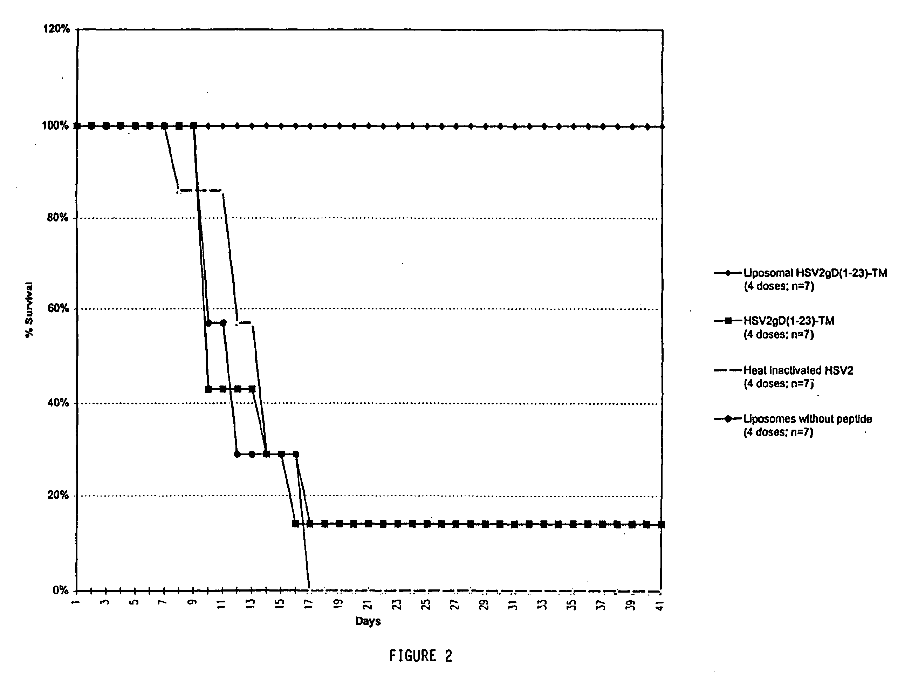

Figure 2 shows a comparison of liposomal HSV2gD(1-23)-TM with other methods of antigen presentation. Each group of 7 mice were given 4 immunizations before challenge with a lethal dose of HSV2. Again, four inoculations with the liposomal HSV2gD(1-23)-TM vaccine results in 100% protection of the mice from a lethal dose of HSV2. Four immunizations with non-liposomal HSV2gD(1-23)-TM, or liposomes without HSV2gD(1-23)-TM are not able to provide significant protection from the lethal HSV2 challenge. This clearly demonstrates that both the liposome and the engineered protein components must be associated with each other in order to achieve an effective immune response. Of particular significance is the finding that the liposomal HSV2gD(1-23)-TM vaccine provides much better protection than immunizations with heat-inactivated virus because viral-based vaccines are generally believed to provide a good stimulus for an immune response (Desrosiers, 1992, AIDS Res. Hum. Retrovir. 8:1457).

At the end of the study, the mice were sacrificed and the serum collected to characterize the nature of the immune response at this time. The serum of the vaccinated mice was found to contain high titers of antibodies (1:100) that specifically recognized the peptide used to stimulate the immune response and further, caused precipitation of active virus, suggesting that the antibodies recognized an epitope on the surface of the virus. The antibodies also precipitate HSV2gD(1-23)-TM liposomes and not the liposomes without the peptide, suggesting that they recognize the gD epitope of HSV2 specifically.

Table 3 below provides a summary of immunological assays demonstrating that liposomal HSV2gD(1-23)-TM given subcutaneously once a week for four weeks generates a stronger immune response when compared to standard adjuvants. Vaccination with antigen mixed with incomplete Freund's adjuvant or alum were unable to provide the mice with protection from viral challenge. The data obtained from the neutralizing antibody test and the delayed hypersensitivity response paralleled the survival data with the highest antibody titer (B cell response) and the greatest amount of footpad swelling (T cell response) observed for the liposomal HSV2gD(1-23)-TM group. Another important advantage associated with the liposomal HSV2gD(1-23)-TM treatment was that, unlike the results in the mice given incomplete Freund's adjuvant with the antigen, there was no irritation or inflammation at the site of injection in any of the mice (17/17 experienced reddening at the injection site with incomplete Freund's adjuvant versus 0/17 for the liposomal HSV2gD(1-23)-TM). When alum was used, 17117 mice developed palpable granulomas at the injection site. In contrast, liposomal HSV2gD(1-23)-TM did not cause the formation of granulomas in any of the mice. In summary, these results suggest that B and/or T cell responses (as judged by neutralizing antibody titers and the degree of footpad swelling) contributes to the protective immune response elicited by liposomal HSV2gD(1-23)-TM in mice against a lethal dose of HSV2.

**Table 3. Summary of results comparing the immunological responses of liposomal HSV2gD(1-23)-TM, incomplete Freund's adjuvant mixed with HSVgD(1-23)-TM. alum mixed with HSV2gD(1-23)-TM, and a control group injected with PBS alone.**

| Vaccine treatment | Surviving mice/# of mice in group- | Neutralizing antibody titer day of challenge | % Footpad swelling relative to control mice |
|---|---|---|---|
| | | | |
| Liposomal HSV2gD (1-23)-TM | 5/7 | 1:1638 | 8% |
| Incomplete Freund's adjuvant with HSV2gD(1-23)-TM antigen | 0/7 | 1:717 | 3% |
| Alum mixed with HSV2Gd(1-23)-TM | 0/7 | 1:1024 | 5% |
| Phosphate buffered saline | 0/7 | 1:486 | 0% |

### Example II - HSV2

*Preparation of HSV2gD(1-23)-HD*. A gene construct encoding HSV2gD(1-23)-HD was generated using overlapping oligonucleotides encoding the gD epitope linked to a 45-amino acid hydrophobic domain (HD). The construct was built by successive elongation and amplification of the coding region of the gene. The gene was then ligated into a pTrcHis expression vector and verified by sequence analysis. A small-scale expression study was initiated by induction with I mM IPTG and confirmed by Western blot analysis using the mouse antibodies generated in vaccination studies with liposomal HSV2gD(1-23)-TM. The protein was also analyzed by SDS-PAGE and a band found that corresponded with the expected molecular weight (≅6kD) as judged by Coomassie staining. Having demonstrated that the expected protein was expressed on a small scale, a ten-liter batch fermentation of *E. coli* containing the pTrcHis/HSV2gD(1-23)-HD construct was completed which yielded approximately 60 grams of cell paste. Following induction, expression of the HSV2gD(1-23)-HD was verified by a Western blot of the fermentation time course. The bacteria from approximately 30 grams of cell paste were lysed by French Press in a buffer containing 1% deoxycholate and 5 mM imidazole. After centrifugation to pellet the cellular debris, the HSV2gD(1-23)-HD was found predominantly in the aqueous soluble supernatant.

Purification was achieved by passing the supernatant containing the soluble HSV2gD(1-23)-HD protein over a nickel-charged chelating Sepharose column. The column was washed successively with solutions containing 5 mM imidazole, 200 mM imidazole, and then 5 mM imidazole with 1% deoxycholate to remove all residual proteins. The HSV2gD(1-23)-HD protein was finally eluted with a 200 mM imidazole, 1% deoxycholate solution. The peak fractions were identified by Coomassie stained SDS-PAGE and by Western blot. All peak fractions containing HSV2gD(1-23)-HD were pooled and concentrated using a Millipore Ultraprep concentrator. The final concentrate was shown to contain pure HSV2gD(1-23)-HD, as judged by the presence of a single band on a Coomassie stained SDS-PAGE gel. From this first run, approximately 2-3 mg of HSV2gD(1-23)-HD was obtained for liposome formulation and vaccination studies.

The liposomal preparation and vaccination procedures, the immune response induction testing, the HSV2 mouse encephalitis model, the antigen-induced T cell proliferation, the cytokine-specific mRNA analysis, the ELISA cytokine measurements, and the anti-HSV2 CTL responses were essentially as described in Example I.

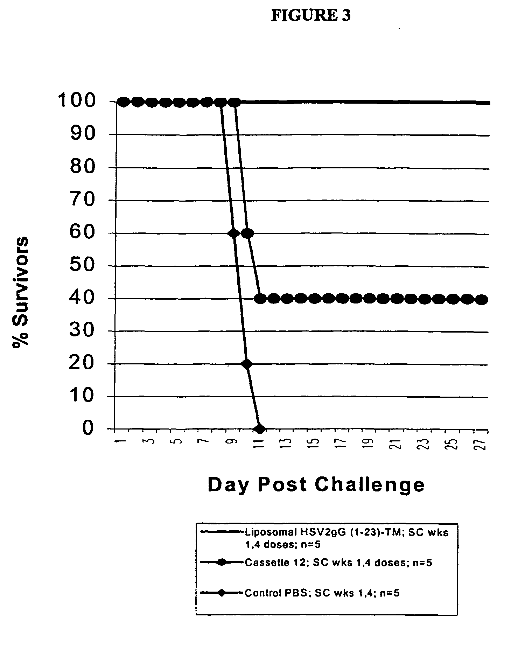

*Results.* The survival curve displayed in Figure 3 shows the ability of the HSV2gD(1-23)-HD to elicit a protective immune response. The % survival with this particular formulation was observed to be 40%. In comparison, buffer treated control animals showed no survivors, while the HSV2gD(1-23)-TM control group had 100% survival.

### Example III - HIV

*Preparation of HIV (gp120-HD) and HIV (*Δ *gp120-HD) in COS-1 cells.* For the conformational epitope study, the gp120 sequence is obtained from the pHXB2-env plasmid containing the gp160 gene (NIH AIDS Research and Reagent Program. Rockville, MD) by amplifying the plasmid DNA by PCR and subcloning the 1536-bp product for sequencing by the dideoxy nucleotide method. Using a 5' (sense) primer corresponding to the first 21-bp of the HIVgp120 gene *ATGAGAGTGAAGGAGAAATAT* and a 3' (antisense) primer corresponding to nucleotides 1515 to 1536 *TGCTCTTTTTTCTCTCTGCAC,* eliminating the HIV gp41 protein, the gp120 segment is cloned and amplified by PCR. In addition, each primer is flanked with convenient restriction enzyme sites to match the multiple cloning sites of the pcDNA4-His expression vector. The PCR product is ligated into the pcDNA4-His containing the HD domain as a gene cassette plasmid (Invitrogen, Inc., San Diego, CA). The deletion mutants are made by PCR amplification using two 5' and 3' primer pairs that exclude nucleotides encoding residues in the gp 120 protein; these include Δ136-151gp120, Δ128-194gp120 and Δ123-203gp120. The primers contain convenient restriction enzyme sequences allowing for ligation of the two gene products. Each mutation is therefore replaced with two amino acids encoded by the specific restriction site of interest. The final genes are verified by sequencing the DNA by using the protocol and reagents from the AutoCycle kit (Amersham Pharmacia Biotech, Piscataway, NJ) and results run on the ALFExpress kit (Amersham Pharmacia Biotech, Piscataway, NJ) sequencing apparatus and analyzed with AM software v.3.02 kit (Amersham Pharmacia Biotech, Piscataway, NJ). The plasmids are transfected into COS-1 cells (ATCC, Rockville, MD) using the DEAE-dextran reagent. Stable transfectants are selected using Zeocin (Invitrogen, Inc., San Diego, CA) and cloned by limiting dilution. The gp120-HD proteins are purified by selective affinity to nickel bound to a solid support. Clones that produce high levels of protein are assessed by Western blot analysis or FACS analysis. The histidine linker of HIV(gp120)-HD, HIVΔ136-151gp120, HIVΔ128-194gp120 and HIVΔ123-203gp120 are removed by enterokinase cleavage after purification is complete. If necessary, size exclusion, ion exchange, or hydrophobic interaction chromatography is performed to further purify the HIVgp120-HD and HIV Δgp120-HD proteins.

*Preparation of HIV-HD*. The nucleotide sequence combining three epitopes (CTL, T helper cell, and B cell) is selected from sequences discovered in different HIV proteins (KQIINMWQEVGKAMYACTRPNYNKRKRIHIGPGRAFYTTK (SEQ ID NO. 19)) and are derived from this protein sequence using *E. coli* codon preferences (Looman et al., 1987,EMBO J. 6:2489). The synthetic gene encoding the HIV sequence is assembled by synthesis, hybridization, PCR, and ligation of overlapping oligonucleotides. The assembled full length gene encoding the HIV fragments is amplified by PCR and then ligated into an expression plasmid. The final genes are verified by sequencing the DNA.

The HIV-HD containing pTrcHis plasmids are transformed into *E. coli.* The bacteria are incubated in LB medium with ampicillin (50 µg/ml) at 37°C until mid-log phase. At this time, IPTG is added to induce the trp/lac hybrid promoter. Hourly time points are examined to determine the optimal post-induction expression of HIV-HD proteins. At the time of optimal expression, cells are harvested by centrifugation. The cell pellets are lysed and centrifuged. The lysate is assayed for the presence of protein. The HIV-HD proteins are purified by selective affinity to nickel bound to a solid support. If necessary, size exclusion, ion exchange or hydrophobic interaction chromatography can be done to further purify the HIV-HD.

*Liposome preparation.* The liposomes are prepared by probe sonication according to previously described procedures (Fujii et al, 1997, Biochemistry 36:4959). Briefly, the lipids (with or without protein) are dissolved in an organic solvent such as chloroform/methanol. Thin lipid films are created by pipetting aliquots of the lipid solutions into round bottom glass tubes, and evaporating the solvent at 65°C under a stream of nitrogen gas. The films are placed under vacuum for at least eight hours to remove residual organic solvent. Preparation of the liposomes is accomplished by hydrating the lipid films with buffer and incubating the suspension at 65°C for 5-10 minutes before probe sonication. The liposomes are then filtered through a 0.22 µm filter to sterilize the preparation. The liposomes are sized by dynamic light scattering and the formation of aggregates followed for at least four weeks.

*Vaccination procedures and testing for induction of immune response to viral antigen*. BALB/c female mice (6-8 weeks old) are subcutaneously vaccinated at weeks 1, 4 and 8 with the test vaccine preparation or buffer. The sites of injection are monitored for adverse reactions such as the development of granulomas and/or scab formation. When the animals are immunized with the vaccine via the intranasal route, they are anesthetized with halothane and the test article (10-20 µl) administered to the nares for inspiration using a sterile tip on a micropipettor (Gallichan et al., 1993, J. Inf. Dis. 168:622).

*Measurement of antibody responses by ELISA.* At regular intervals during the vaccination procedure, (e.g., 1 x per week), the immune response of the mice to the viral antigen is measured. Liposomes containing the antigenic epitope are also used as targets for an antibody/ELISA assay as described earlier (Tuso et al., 1993, Transplantation 55:1375). The results of the latter assay are used to measure the level of binding and isotype configuration of the antibodies.

*Delayed type hypersensitivity (DTH) assay*. A DTH response to the viral antigen in vaccinated mice is tested using a footpad assay. Vaccinated animals are anesthetized with halothane and injected with 50 µl of either liposomal antigen or free antigen in one hind footpad and 50 µl buffer in the other hind footpad. Twenty-four, forty-eight and seventy-two hours later, the degree of footpad swelling of the mice is measured using a Vernier caliper and compared to the baseline measurements.

*Cytokine-specific mRNA Analysis.* T cells from immunized and non-immunized animals are examined for cytokine-specific mRNA levels for the major cytokines that may reflect sensitization associated with immunization, including IL-2, IFN-γ, IL-4, IL-5, IL-6, and IL-10. Splenic T cells are isolated as described previously at 1 and 4 weeks following immunization. Total RNA is isolated using a modification (Cosenza et al., 1995, Liver Transpl. Surg. 1:16) of a method described by Chomczynsky et al., 1987, Analyt. Biochem. 162:156. Fresh or frozen cells (2 x 10⁶) are disrupted in 1 mL of denaturing solution (4 mM guanidinium thiocyanate, 25 mM sodium citrate (pH 7.0), 0.5% sarcosyl, and 0.1 mM 2-mercaptoethanol). The RNA is precipitated by incubating with one volume of isopropanol overnight at -20°C. The concentration of total RNA is adjusted to 260 nM and the samples stored at -80°C until analyzed.

Cytokine analysis is conducted using a modification (Cosenza et al., 1995, Liver Transpl. Surg. 1:16) of a method described by Chomczynsky et al., 1987, Analyt. Biochem. 162:156. Single strand cDNA is prepared by transcription of 2 µg of total RNA in 20 µl of total reaction mixture using avian myeloblastosis virus reverse transcriptase (Boehringer Mannheim, Indianapolis, IN). Sequence specific oligonucleotide primers for murine cytokines (Table 1) are used to amplify DNA fragments of predetermined size for each of the cytokine genes of interest. The PCR mixture consists of 1 µl of cDNA, 2.5 µl of PCR 10x buffer, 1 µl of each 5' and 3' primers, 2.5 µl of 10x dNTPs (2 mmol/L) and 0.125 µl of *T. aquaticus* thermostable DNA polymerase (Boehringer Mannheim) in a final volume of 25 µl. Specific β-actin primers are used to amplify a 548 bp fragment as an internal control. The PCR mixture is covered with mineral oil and amplification conducted following incubation of the mixture for 7 minutes at 94°C, 38 cycles with 30 second denaturation at 94°C, 45 second primer annealing at 60°C, 60 second extension at 72°C, and a 7 minute incubation at 72°C. The PCR products are separated on agarose gel in the presence of ethidium bromide. Bands are visualized under UV light, photographed, and densitometrically quantified from the photograph using the Molecular Analyst software package (Bio-Rad, Richmond CA).

*Cytokine measurement by ELISA in splenic T cells.* The T cell activity of these animals is also measured by the production of cytokines in response to incubation of splenic T cells with viral antigen. The spleens are removed from the vaccinated animals and spleen homogenates prepared by gently breaking up the spleens with a sterile plunger and pushing the cells through a nylon screen mesh. The cell suspensions are rinsed and plated at 1x10⁵ cells/well in a 96-well microtiter plate. Following incubation of the cells at 37°C for 3 to 4 days with different amounts of viral antigen, the supernatants from the wells are harvested and tested for the presence of cytokines. Commercially available cytokine ELISA kits are used to define the cytokine profile. Detection of the cytokine protein in tissue culture supernatants by sandwich ELISA is performed by using monoclonal antibodies (mAb) specific for the particular cytokines. Briefly, ELISA plates are coated overnight with a rat anti-mouse cytokine mAb. Plates are blocked with 3% fetal calf serum in PBS for 2 hours, then aliquots of each test sample are added to each well. Cytokine specific biotinylated rat anti-mouse mAb is added to each well followed by avidin peroxidase. A color reaction is achieved by the addition of colorimetric substrates. Plates are read in an ELISA spectrophotometer and cytokine concentrations calculated from a standard curve obtained from control recombinant cytokines. All cytokine ELISA kits (IL-2, IL-4, IL-6, IL-10, IL-12, IFN-γ, and TNF-α) are purchased from PharMingen, Inc. (San Diego, CA).

*Antigen-induced T cell proliferation.* Sensitization of the host to the HSV antigens is measured using antigen-induced T cell proliferation. Spleen homogenates are isolated as described previously. The cells are suspended in RPMI 1640 medium containing 10% FCS, 10 mM Hepes buffer, L-glutamine (2 mM), penicillin (25 IU/ml), streptomycin (25 µg/ml), and gentamycin (80 µg/ml). The cells are cultured at a concentration of 1 x 10⁶ cells/ml with or without the IUV-HD peptide (5 µg/ml) in 96-well U-bottom plates for 3-4 days in 5% CO₂. The cultures are pulsed with 20 µl of resazurin dye for 3 hours and then the fluorescence is measured on the Cytofluor II (Perseptive Biosystems, Inc., Farmington, MA).

*Anti-HIV CTL responses.* After vaccination, the presence of antigen-specific cytotoxic T lymphocytes (CTL) in splenic T cells are assessed. Splenic lymphocytes are obtained as described previously. Lymphocytes from each treatment group are pooled (n=5) and then cultured for 3 days without antigen at 10⁷ cells/well in 12-well culture plates. L5198Y lymphoma (H2*^{d}*) target cells (ATTC) are incubated with a peptide corresponding to the HIV CTL epitope for H2*^{d}* restricted cells and incubated for 4 hours at 37 °C. Targets are washed, and 100µl titers containing 1 x 10⁴ cells are added to each well. Effector lymphocytes are washed, added to wells at various concentrations and cultured for 4 hours at 37 °C. Using the CytoTox 96 kit (Promega, Madison WI), the percent specific lysis is calculated from supernatant lactate dehydrogenase (LDH) measured in a standard ELISA plate reader (HTS 7000+ BioAssay Reader, Perkin Elmer, San Jose, CA) by recording the absorbance at 490 nm. The determination of HSV2-antigen specific lysis is made according to standard criteria. Data are expressed as percent specific lysis = 100 x [(experimental -effector spontaneous - target spontaneous)/(target maximum - target spontaneous)].

### Example IV - Influenza Virus (INFV)

*Preparation of INFV-HD*. The nucleotide sequence of the chosen epitopes is derived from the protein sequence using *E. coli* codon preferences (Looman et al., 1987, EMBO J. 6:2489). The antigen sequence(s) inserted into the gene cassette corresponds to the epitopes from NP (147-161, TYQRTRALVRTGMDP; 55-69 (SEQ ID NO. 20), RLIQNSLTIERMVLS (SEQ ID NO. 21)) and HA (91-108, SKAFSNCYPYDVPDYASL (SEQ ID NO. 22)). A combination epitope vaccine can be constructed consisting of a T-B epitope as reported by Brumeanu et al., 1997 (HA 110-120/HA 150-159, SFERFEIPKEGGWLTEKEGYSP (SEQ ID NO. 23)) (Brumeanu et al., 1997, J. Virol. 71:5473). Once the appropriate sequence(s) are obtained, a synthetic gene encoding the INFV-HD is assembled by synthesis, hybridization, PCR, and ligation of overlapping oligonucleotides. The assembled full length gene encoding the INFV fragments is amplified by PCR and ligated into an expression plasmid. The final genes are verified by sequencing the DNA.

The INFV-HD containing plasmids are transformed into *E. coli.* The bacteria are incubated in LB medium with ampicillin (50 µg/ml) at 37°C until mid-log phase. At this time, IPTG is added to induce the *trp*/*lac* hybrid promoter. Hourly time points are examined to determine the optimal post-induction expression of INFV-HD proteins. At the time of optimal expression, cells are harvested by centrifugation. The cell pellets are lysed and centrifuged. The lysate is assayed for the presence of protein. The INFV-HD proteins are purified by selective affinity to nickel bound to a solid support. If necessary, size exclusion, ion exchange, or hydrophobic interaction chromatography are performed to further purify the INFV-HD.

*Viral infection model.* The INFV strain used to demonstrate the efficacy of the vaccine is a reassortant of the A/PR/8/34 virus called PR8. The virus is cultured in 10 to 12 day old embryonated chicken eggs by allantoic inoculation, followed by incubation at 37°C in a rocker for 40-48 hours and 20-24 hours at 4°C, and then recovered from the allantoic fluid. A viral hemagglutination assay with 0.5% chicken red blood cells will be conducted in 96-well microtiter dishes to determine the hemagglutinating units (HAU)/ml of the viral stock.

In the BALB/c mouse model, it was shown that 1200 HAU of the PR8 strain, administered intranasally with a sterile tip of a micropipettor to metofane anesthetized mice (20µl), produced pronounced symptoms of infection by day 4, including decreased mobility, lack of grooming, a 20% loss of body weight and death within two weeks. Using a MDCK cytolysis assay conducted in 96-well microtiter dishes with a 50% endpoint, a high titer of infectious virus was found to be present in lung homogenates prepared 4 days post-infection."

*Vaccination procedures and testing for induction of immune response to viral antigen*. BALB/c female mice (6-8 weeks old) are subcutaneously injected at weeks 1, 4 and 8 with the vaccine preparation or buffer. The sites of injection are monitored for adverse reactions such as the development of granulomas. When the animals are immunized with the vaccine via the intranasal route, they are anesthetized with metofane, and the test article (10-20 µl) administered to the nares for inspiration using a sterile tip on a micropipettor (Gallichan et al., 1993, J. Inf. Dis. 168:622).

*RT-PCR detection of INFV RNA in lung tissue.* RT-PCR detection of INFV RNA is conducted on the lungs of experimental animals taken 4 days after viral challenge to establish the effectiveness of immunization for preventing viral infection. Lung samples taken from the mice are snap-frozen, ground, and the frozen powder stored at -70°C until processed for evaluation. Total RNA isolation and RT-PCR analysis for viral RNA is conducted using a modification of previously described techniques (Chomczynsky et al., 1987, Analyt. Biochem. 162:156; Shirwan et al., 1993, J. Immunol. 151:5228; Lakeman and Whitley, 1995, J. Inf. Dis. 171:857). The primers to be used are specific for the matrix gene (segment 7) of influenza virus A (Atmar et al., 1996, J. Clin. Microbiol. 34:2604). The RT-PCR assay is conducted using the Titan One Tube RT-PCR System (Boehringer Mannheim, Indianapolis, IN) reagents and protocol. Briefly, 1 pg to 1 µg total RNA template is incubated in the presence of 0.2 mM dNTPs, 0.4 µM antisense primer FAM1 (5'-CAGAGACTTGAAGATGTCTTTGC-3' (SEQ ID NO. 24)), 0.4 µM sense primer, FAM2 (5'-GCTCTGTCCATGTTATTTGGA-3' (SEQ ID NO. 25)), 5 mM DTT, 5 U RNase inhibitor, 1.5 mM MgCl₂, and AMV/Expand High Fidelity enzymes under the following conditions: one cycle of 60°C for 30 minutes; 94°C for 2 minutes; ten cycles of 94°C for 30 seconds, 55°C for 30 seconds, 68°C for 45 seconds; twenty-five cycles of 94°C for 30 seconds, 55°C for 30 seconds, 68°C for 45 seconds increasing extension five seconds for every cycle; followed by one cycle of 68°C for 7 minutes. A positive result is based upon a visible band of 212 bp in a 1.5% NuSieve-agarose gel (FMC Bioproducts, Rockland, ME) stained with ethidium bromide and photographed with UV transilluminator (BioRad Gel Doc 1000). This method has been shown to be sensitive and highly specific for measuring the presence of viral DNA (Lakeman and Whitley, 1995, J. Inf. Dis. 171:857).

*Sequence of the HA1 domain.* The HA1 domain (segment 4) sequence can be elucidated by amplifying the HA1 domain from viral RNA in a RT-PCR reaction, then the 1100 bp product subcloned for sequencing, and sequenced using the dideoxy-nucleotide termination method. The RT-PCR assay is conducted using the Titan One Tube RT-PCR System as noted above with the following differences; the amplification primers are the cDNA primer (5'-AGCAAAAGCAGGGGAAAATAAAAAC-3' (SEQ ID NO. 26)) and PCR primer (5'-CAATGAAACCGGCAATGGCTCC-3' (SEQ ID NO. 27)) (Pyhala et al., 1995 J. Gen. Virol. 76:205), the conditions will be one cycle of 60°C for 30 minutes; 94°C for 2 minutes; ten cycles of 94°C for 30 seconds, 60°C for 30 seconds, 68°C for 45 seconds; twenty-five cycles of 94°C for 30 seconds, 60°C for 30 seconds, 68°C for 45 seconds increasing extension five seconds for every cycle; followed by one cycle of 68°C for 7 minutes. The 1100 bp product is subcloned into the TA PCR 2.1 cloning vector (Invitrogen, Carlsbad, CA) for subsequent sequencing. Sequencing is carried out using the protocol and reagents from the AutoCycle kit (Amersham Pharmacia Biotech, Piscataway, NJ) and results run on the ALFExpress kit (Amersham Pharmacia Biotech, Piscataway, NJ) sequencing apparatus and analyzed with AM software v.3.02kit (Amersham Pharmacia Biotech, Piscataway, NJ).

*Measurement of antibody responses by ELISA.* At regular intervals during the vaccination procedure, the immune response of the mice to the viral antigen is measured. Liposomes containing the antigenic epitope are also used as targets for an antibody/ELISA assay as described earlier (Tuso et al., 1993, Transplantation 55:1375). The results of the latter assay are used to measure the level of binding and isotype configuration of the antibodies.

*Antigen-induced T cell proliferation.* Sensitization of the host to the INFV antigens are measured using antigen-induced T cell proliferation. Spleens are removed from vaccinated mice and homogenates prepared by gently breaking up the spleens with a sterile plunger and pushing the cells through a nylon screen mesh. The cells are suspended in RPMI 1640 medium containing 10% FCS, 10 mM Hepes buffer, L-glutamine (2mM), penicillin (25 IU/ml), streptomycin (25 µg/ml), and gentamycin (80 µg/ml). The cells are cultured at a concentration of 1 x 10⁶ cells/ml with or without chemically synthesized peptides corresponding to the antigens (5 µg/ml) in 96-well U-bottom plates for 3-4 days in 5% CO₂. The cultures are pulsed with 20 µl of resazurin dye for 3 hours and then the fluorescence is measured on the Cytofluor II (Perseptive Biosystems, Inc., Farmington, MA).

*Anti-INFV CTL responses.* After vaccination, the presence of antigen-specific cytotoxic T lymphocytes (CTL) in splenic T cells are assessed. Splenic lymphocytes are obtained as described previously. Lymphocytes from each treatment group are pooled (n=5) and then cultured for 3 days without antigen at 10⁷ cells/well in 12-well culture plates. P815 mastocytoma or L5178 lymphoma (H2*^{d}*) target cells (ATTC) are incubated with a peptide corresponding to the INFV NP CTL epitope (amino acids 147-158) for H2*^{d}* restricted cells and incubated for 4 hours at 37 °C. Targets are washed, and 100µl titers containing 1 x 10⁴ cells are added to each well. Effector lymphocytes are washed, added to wells at various concentrations and cultured for 4 hours at 37 °C. Using the CytoTox 96 kit (Promega, Madison WI), the percent specific lysis is calculated from supernatant lactate dehydrogenase (LDH) measured in a standard ELISA plate reader (HTS 7000+ BioAssay Reader, Perkin Elmer, San Jose, CA) by recording the absorbance at 490 nm. The determination of HSV2-antigen specific lysis is made according to standard criteria. Data are expressed as percent specific lysis = 100 x [(experimental -effector spontaneous - target spontaneous)/(target maximum - target spontaneous)].

### Example V - Nontypable H. influenzae (NTHi) and H. influenzae type b (Hib)

*Cloning of the NTHi proteins.* The full length gene sequence encoding P6 is obtained from a strain of NTHi by RT-PCR amplification of the P6 mRNA (Deich et al, 1988, J. Bacteriol. 170:489; Nelson et al., 1988, Inf. Immun. 56:128; Looman et al., 1987, EMBO J. 6:2489). Amplification is conducted using the Titan One Tube RT-PCR System (Boehringer Mannheim, Indianapolis, IN) reagents and protocol. Briefly, bacteria are incubated in the presence of 0.2 mM dNTPs, 0.4 µM Antisense primer H-InvfP6AS (5'GAGTCCGCTGCTCTACCAAC -3' (SEQ ID NO. 28)), 0.4 µM Sense primer, H-InvfP6S (5'-AATTTCCAGCTTGGTCTCCA -3' (SEQ ID NO. 29)), 5 mM DTT, 5 U RNase inhibitor, 1.5 mM MgCl₂, and AMV/Expand High Fidelity enzymes under the following conditions: one cycle of 60°C for 30 minutes; 94°C for 2 minutes; ten cycles of 94°C for 30 seconds, 55°C for 30 seconds, 68°C for 45 seconds; twenty five cycles of 94°C for 30 seconds, 55°C for 30 seconds, 68°C for 45 seconds, increasing extension five seconds for every cycle; followed by one cycle of 68°C for 7 minutes. A positive result is based upon a visible band of 867 bp in a 1.0% NuSieve-agarose gel (FMC Bioproducts, Rockland, ME) stained with ethidium bromide and photographed using a digital image system (BioRad, Gel Doc 2000). The PCR product is subcloned for sequencing purposes into the TA 2.1 Vector using the TA Cloning kit (Invitrogen, Carlsbad, CA) reagents and protocol. A small scale plasmid preparation is performed using the standard alkaline lysis method (Maniatis, 1989 Molecular Cloning: A Laboratory Manual, 2nd Ed., Sambrook, Fritsch, Maniatis, Cold Spring Harbor Laboratory Press, NY, p.125). The PCR product insert is sequenced by the conventional dideoxy termination method using the protocol and reagents from the AutoCycle kit (Amersham Pharmacia Biotech, Piscataway, NJ) and results run on the ALFExpress II (Amersham Pharmacia Biotech, Piscataway, NJ) sequencing apparatus and analyzed with AlfWin Sequence Analyser software v.2.0 (Amersham Pharmacia Biotech, Piscataway, NJ).

*Preparation of NTHi(P6)-HD.* Once the gene encoding P6 is obtained, a synthetic gene encoding the NTHi(P6)-HD is assembled by synthesis, hybridization, PCR, and ligation of overlapping oligonucleotides. The HD segment is added to either the N- or C-terminus of the protein via a linker composed of glycine and serine. This allows us to test the effect that the relative location of the HD with respect to the antigen has on processing by the immune system. The assembled full length gene encoding the NTHi(P6)-HD fragment is amplified by PCR and ligated into an expression plasmid. The final gene is verified by sequencing the DNA.

*The NTHi(P6)-HD containing plasmid is transformed into E. coli.* The bacteria is incubated in LB medium with ampicillin (50 µg/ml) at 37°C until mid-log phase. At this time, IPTG is added to induce the trp/lac hybrid promoter. Hourly time points are examined to determine the optimal post-induction expression of NTHi(P6)-HD protein. At the time of optimal expression, cells are harvested by centrifugation. The cell pellets are lysed and centrifuged. The lysate and cell pellet are assayed for the presence of protein. A 1-2% solution of sodium deoxycholate is used to extract the NTHi(P6)-HD protein from the lysed cell pellet. The NTHi(P6)-HD is then purified by selective affinity to nickel bound to a solid support. If necessary, size exclusion, ion exchange, or hydrophobic interaction chromatography can be done to further purify the NTHi(P6)-HD.

*Liposome preparation.* There are three general approaches to prepare stable NTHi(P6)-HD liposomes. In the first, the liposomes are formulated such that the protein and lipids are dissolved together in the organic solvent (chloroform/methanol) and then dried into a thin film. Upon resuspension in aqueous buffer, the lipids and protein assemble into large bilayer structures. The suspension is then sonicated to make small unilamellar vesicles (SUV). For the smaller protein designs, this method works well. However, for these experiments, it may not be desirable to expose the protein to harsh solvent conditions because of the potential to denature the protein. Thus, to avoid undesirable conformational changes, a second method can be used that involves solubilizing NTHi(P6)-HD in the resuspension buffer prior to hydration of the lipid film. Upon hydration with the lipids, the protein spontaneously associates with the newly formed membrane and becomes incorporated into the bilayer of the SUV during sonication. The third method involves preparing the liposomes without the NTHi(P6)-HD protein and then adding it to the already formed SUV, allowing the HD to integrate into the lipid bilayer. The liposomes are prepared by probe sonication according to previously described procedures (Fujii et al., 1997, Biochemistry 36:4959). Briefly, the lipids (with or without protein) are dissolved in an organic solvent such as chloroform/methanol. Thin lipid films are created by pipetting aliquots of the lipid solutions into round bottom glass tubes, and evaporating the solvent at 65°C under a stream of nitrogen gas. The films are placed under vacuum for at least eight hours to remove residual organic solvent. Alternatively, lipid powders of the desired composition may be prepared by a technique such as spray drying and then used in place of the lipid films. Preparation of the liposomes is accomplished by hydrating the lipid films or powders in buffer and incubating the suspension at 65°C for 5-10 minutes before probe sonication. The liposomes are then filtered through a 0.22 µm filter to sterilize the preparation. The liposomes will be sized by dynamic light scattering and the formation of aggregates followed for at least four weeks. It is possible that the conformation of the NTHi(P6)-HD protein may need to be conserved. Therefore, of the three approaches that may be used to prepare the liposomes, we anticipate that either addition of the protein as a component of the hydrating solution or addition to the liposomes after they have already been formed may be preferred.

*Bacterial strains.* NTHi strains 9333 (spinal fluid isolate), 35056 (upper respiratory infection isolate), 43095 (otitis media isolate), 49766 (lung abscess isolate, reference strain for antimicrobial susceptibility testing) (ATCC) and Hib strain 51654 (ATCC) were used to demonstrate efficacy in the bacterial infection model. Prior to use, a frozen stock of the organism is subcultured in fresh Brain Heart Infusion broth supplemented with NAD (2 µg/ml) and heme (10 µg/ml), and incubated for 24 hours at 37°C with 10% carbon dioxide. A standard curve for each organism consisting of colony forming units versus turbidity at 480 nm is used to adjust the inoculum of bacteria needed for either the bactericidal assays or the intraperitoneal challenge dose.

*Vaccination procedures and testing for induction of immune response to bacterial antigen*. Groups of infant rats (n=6 per group) are immunized on post-natal days 5, 12, 19, and 26 or on days 5 and 26 with the test vaccine preparation or buffer. Systemic vaccination of the rats is done by subcutaneous injection of the liposomal vaccines. When the animals are immunized with the vaccine via the intranasal route, they are anesthetized with metofane, and the test article (20 µl) is administered using a sterile tip on a micropipettor (Gallichan et al., 1993, J. Inf. Dis. 168:622). The infant rats are housed with their nursing mothers and monitored for adverse reactions to the vaccination procedure, such as the formation of granulomas, reddening or scabbing at the site of inoculation. One week after the last vaccination, the infant rats are anesthetized with halothane and the blood is collected by cardiac puncture and the mucosal passages lavaged with saline for collecting mucosal secretions. The animals are euthanized with carbon dioxide. The bactericidal antibody titers of the serum and mucosal fluid is measured as described below.

For the intraperitoneal challenge study, the infant rats (26 days old) are challenged with the bacteria and their blood and CSF monitored for bacterial burden compared to unvaccinated rats. Sampling of the blood and the CSF for bacteria two days post-challenge indicates that there is a significant bacterial burden in the animals (*i*.*e*., 1x10⁴ CFU/ml blood; 1x10⁴ CFU/ml CSF). The serum from the vaccinated 26-day old rats containing high titers of bactericidal antibodies is administered intravenously to 6-day old rats. The next day, infant rats (n=10) receive an intraperitoneal dose ofNTHi as described below. The infant rats are then monitored for morbidity and mortality, survivors are euthanized after 26 days, and the blood and CSF collected and cultured for the presence of NTHi.

*Intraperitoneal challenge with NTHi.* A 24-hour old culture of *in vivo* passaged NTHi is adjusted to 5x10⁷-5x10⁹ CFU/ml and 100 µl of this culture injected intraperitoneally into 5-10 day old rats (Smith et al., 1973, Inf. Immun. 8:278). Challenged infant rats remain with the nursing mothers after inoculation. Within 18-96 hours post-challenge, at least 90% of the infant rats die using this dose of passaged bacteria.

*Measurement of antigen specific antibody responses by ELISA.* Serum and mucosal samples from each rat are tested for IgG and IgA antibodies specific for the P6 antigen. Either the cloned native P6 or the NTHi(P6)-HD protein is added to appropriately treated 96 well plates for overnight incubation at room temperature. Plates are blocked with 1% bovine serum albumin in bicarbonate buffer, pH 9.6 for 30 minutes at 37°C. Plates are then rinsed with 0.05% Tween 20/PBS. Dilutions of each serum and mucosal sample are added to the antigen-coated plates followed by incubation for 2 hours at 37°C and rinsing with Tween/PBS buffer at the end of the incubation. Alkaline phosphatase linked monoclonal antibodies specific for rat IgG or IgA are added to the wells for one hour at 37°C. The wells are then rinsed with Tween/PBS buffer and PNPP substrate in diethanolamine buffer, pH 9.5, added to the wells to initiate the colorimetric reaction. Reactions are arrested with 0.75N sodium hydroxide and read at 405nm in a Spectramax ELISA plate reader. Rat IgG and IgA concentrations are calculated from a standard curve obtained from control samples of known concentrations of rat IgG or IgA coated onto 96 well plates and treated as described above.

*Bactericidal antibody assay.* Serum samples to be tested for the presence of bactericidal antibodies are incubated at 56°C for 30 minutes to inactivate complement. Test sera are then serially diluted in phosphate buffered saline (PBS). A 24 hour bacterial culture is adjusted to 5x10⁴ CFU/ml in sterile PCM buffer composed of PBS with 0.15 mM CaCl₂ and 1 mM MgCl₂ containing bovine serum albumin (BSA). Sera from unvaccinated infant rats is used as the complement source. This sera is pooled, aliquoted and stored at -70°C until use. Bacteria (20 µl), serially diluted serum (20 µl), complement (20 µl) and 40 µl of 1% BSA in PCM buffer is added to each well of a 96-well cluster plate. To determine the initial bacterial concentration, 10 µl aliquots of a sample mixture is plated at time zero onto fresh BHI agar plates containing nutrient agar and supplemented with NAD and heme. Following incubation of the reaction mixtures at 37°C with 10% carbon dioxide for one hour with shaking, 10 µl from each well is plated in duplicate and incubated overnight at 37°C in the presence of 10% carbon dioxide to determine the CFU/well. Controls include a well without serum to ensure that the complement alone is not killing the bacteria, and a well with test serum, but without complement to be sure that the serum complement in the test sample had been successfully inactivated. All assays are done in triplicate and those dilutions producing 50% killing of the bacteria are used to compare the activity between different sera. Example VI- Hepatitis C Virus

*Preparation of HCV(E2*/*NS1)-HD and HCV(E2*/*NS1*Δ*HVR1)-HD in CHO cells.* The nucleotide sequence of the HCV E2/NS1 protein is derived from PCR amplification of a strain of HCV obtained as described below. Using a 5' primer corresponding to the first 21-bp of the E2/NS1 gene CAAACCATGATCGCCCACGGA and a 3' primer corresponding to residues 622 to 628, GAAGTTGACAGTGCAGGGGTA, eliminating the transmembrane domain (Cocquerel, L. et al., 1998, J. Virol. 72(3):2183-91). In addition, each primer is flanked with convenient restriction sites. The PCR product is ligated into the pcDNA3.1His containing the HD domain as a gene cassette plasmid (Invitrogen, Inc., San Diego, CA). HCV(E2/NS1ΔHVR1)-HD is constructed by deleting the HVR1 of the E2/NS1 gene corresponding to first 27 amino acids from residue 384 to residue 410. The deletion mutant is made by PCR amplification using a 5' primer CAACTCATCAACACCAATGGC and the same 3' primer used for the wild type control. The final genes are verified by sequencing the DNA. The plasmids are transfected into CHO cells (Deen, K. C. et al., 1988, Nature 331:82) (ATCC, Rockville, MD) using the lipofection reagent (Lipofectamine, Gibco/BRL Gaithersburg, MD) by the manufacturer's recommended protocol. Stable transfectants are selected using G418 (Gibco/BRL, Gaithersburg, MD) and cloned by limiting dilution. Identification of clones that produce high levels of protein are assessed by western blot analysis. The HCV(E2/NS1)-HD and HCV(E2/NS1ΔHVR1)-HD proteins are purified by selective affinity to nickel bound to a solid support. The histidine linker is removed by enterokinase cleavage after purification is complete. If necessary, size exclusion, ion exchange, and/or hydrophobic interaction chromatography are performed to further purify the HCV(E2/NS1)-HD and HCV(E2/NS1ΔHVR1)-HD proteins.

*Preparation of the HCV(HVR1)-HD and HCV(C)-HD proteins in E. coli.* The nucleotide sequences of the chosen epitopes are derived from the protein sequence using *E*. *coli* codon preferences (Looman et al., 1987, EMBO J. 6: 2489). The antigen sequence(s) to be inserted into the gene cassette corresponds to the epitopes from NP (147-161, TYORTRALVRTGMDP; 55-69, RLIONSLTIERMVLS) and HA (91-108. SKAFSNCYPYDVPDYASL). Once the appropriate sequence(s) are obtained, a synthetic gene encoding the HCV-HD, flanked by convenient restriction sites, is assembled by synthesis, hybridization, and ligation of overlapping oligonucleotides. Briefly, assembly of the fragments is achieved by heating to 65°C and annealing the oligonucleotides as they return to room temperature. Following incubation for 2 minutes on ice, the fragments are ligated with DNA ligase. The assembled full length gene encoding the HCV fragments is amplified by PCR, cleaved by restriction enzymes and isolated by gel electrophoresis. The HCV gene is then ligated into a similarly cleaved expression plasmid containing the HD gene (e.g., pTrcHis, or pQE30). The final genes are verified by sequencing the DNA.

The HCV-HD containing plasmids are transformed into *E. coli*. The bacteria are incubated in LB medium with ampicillin (50 µg/ml) at 37°C until mid-log phase. At this time, IPTG is added to induce the *trp*/*lac* hybrid promoter. Hourly time points are examined to determine the optimal post-induction expression of HCV-HD proteins. At the time of optimal expression, cells are harvested by centrifugation. The cell pellets are lysed and centrifuged. The lysate is assayed for the presence of protein. The HCV-HD proteins are purified by selective affinity to nickel bound to a solid support. The histidine linker is removed by enterokinase cleavage after purification is complete. If necessary, size exclusion, ion exchange, or hydrophobic interaction chromatography are performed to further purify the HCV-HD.

The liposomal preparation and vaccination procedures, the immune response induction testing, the ELISA antibody response measurements, the DTH assay, the cytokine-specific mRNA analysis, the ELISA cytokine measurements, the antigen-induced T cell proliferation, and the anti-HIV CTL responses are essentially as described in Example III above.

## Claims

1. An immunogenic liposome composition comprising:
vesicle-forming lipids; and
an antigenic protein construct which is an aqueous soluble fusion product comprising one or more antigen determinants and a hydrophobic domain, wherein the hydrophobic domain is associated with the membrane of the immunogenic liposome composition.

2. An immunogenic liposome composition according to claim 1, further comprising one or more adjuvants.

3. An immunogenic liposome composition according to claim 1, wherein the antigenic construct is chemically synthesized.

4. An immunogenic liposome composition according to claim 1, wherein the hydrophobic domain is a peptide.

5. An immunogenic liposome composition according to claim 4, wherein the hydrophobic domain consists of from about 15 to about 500 amino acid residues.

6. An immunogenic liposome composition according to claim 5, wherein the hydrophobic domain comprises one or more amino acids selected from the group consisting of Ala, Asn, Cys, Gln, Gly, His, Ile, Leu, Met, Phe, Pro, Ser, Thr, Trp, Tyr and Val.

7. An immunogenic liposome composition according to claim 1, wherein the antigenic determinant is derived from an antigen selected from the group consisting of HSV gB or gD, HIV gp 120, CMV gB, HCV E2, INFV HA or NA, and H. influenzae P6.

8. An immunogenic liposome composition according to claim 1, wherein the antigenic construct further comprises a linker region linking the one or more antigenic determinants with the hydrophobic domain.

9. An immunogenic liposome composition according to claim 8, wherein the linker region is a peptide.

10. An immunogenic liposome composition according to claim 9, wherein the linker region consists of from 1 to about 200 amino acid residues.

11. An immunogenic liposome composition according to claim 10, wherein the amino acid residues are selected from the group consisting of Ala, Arg, Asn, Asp, Cys, Glu, Gln, Gly, His, Ile, Leu, Lys, Met, Phe, Pro, Ser, Thr, Trp, Tyr and Val.

12. An immunogenic liposome composition of claim 1, comprising an antigenic construct comprising one or more antigenic determinants and a hydrophobic domain, and a pharmaceutically acceptable carrier.

13. An immunogenic liposome composition according to claim 12, wherein the antigenic construct further comprises a linker region linking the one or more antigenic determinants with the hydrophobic domain.

14. An immunogenic liposome composition according to claim 12, further comprising one or more adjuvants.

15. An immunogenic liposome composition of claim 1, wherein the one or more antigenic determinants is an antigenic determinant of a bacterial pathogen or toxin, of a viral pathogen, of a fungal pathogen, or of a parasite pathogen.

16. An immunogenic liposome composition according to any one of the preceding claims for use in a method of immunising of a host animal.

17. An immunogenic liposome composition according to any one of the preceding claims for use in a method of inducing an immunogenic response in a host animal.

18. An immunogenic liposome composition according to claim 16 or 17, wherein the host animal is a mammal.

19. An immunogenic liposome composition according to claim 18, wherein the mammal is a human.

20. An immunogenic liposome composition according to claim 19. wherein the antigenic determinant is selected from the group consisting of HSV gB or gD, HIV gp120, CMV gB, HCV E2, INFV HA or NA, and H. influenzae P6.

21. An immunogenic liposome composition according to claim 16 or 17, wherein the animal is a bird.

22. A method of making an immunogenic liposome composition comprising:
expressing a gene that encodes an aqueous soluble fusion protein comprising an antigenic determinant and a hydrophobic domain; and
(a) dissolving vesicle-forming lipids and the fusion protein in a suitable organic solvent;
forming a lipid film or spray dried powder by evaporating the organic solvent;
hydrating the lipid film or powder; and
dispersing the hydrated lipid film or powder to form an immunogenic liposome composition; or
(b) dissolving the fusion protein in an aqueous buffer;
hydrating either a lipid powder or a lipid film with the buffer containing the fusion protein; and
dispersing the lipid powder or film to form an immunogenic liposome composition; or
(c) dissolving the fusion protein in an aqueous buffer; and
adding the fusion protein to pre-formed liposomes to form an immunogenic liposome composition.

23. An immunogenic liposome composition obtainable by a method as claimed in claim 22.

## Patentansprüche

1. Eine immunogene Liposomzusammensetzung, welche vesikelformende Lipide und ein antigenes Proteinkonstrukt enthält, welches ein wasserlösliches Fusionsprodukt ist, das eine oder mehrere Antigendeterminanten und eine hydrophobe Domäne enthält, wobei die hydrophobe Domäne mit der Membran der immunogenen Liposomzusammensetzung assoziiert ist.

2. Immunogene Liposomzusammensetzung nach Anspruch 1, welche darüber hinaus ein Adjuvans oder mehrere Adjuvantien enthält.

3. Immunogene Liposomzusammensetzung nach Anspruch 1, wobei das antigene Konstrukt chemisch synthetisiert ist.

4. Immunogene Liposomzusammensetzung nach Anspruch 1, wobei die hydrophobe Domäne ein Peptid ist.

5. Immunogene Liposomzusammensetzung nach Anspruch 4, wobei die hydrophobe Domäne aus etwa 15 bis etwa 500 Aminosäureresten besteht.

6. Immunogene Liposomzusammensetzung nach Anspruch 5, wobei die hydrophobe Domäne eine oder mehrere Aminosäuren enthält, welche aus der Gruppe, welche aus Ala, Asn, Cys, Gln, Gly, His, Ile, Leu, Met, Phe, Pro, Ser, Thr, Trp, Tyr und Val besteht, ausgewählt sind.

7. Immunogene Liposomzusammensetzung nach Anspruch 1, wobei die antigene Determinante von einem Antigen abgeleitet ist, welches aus der Gruppe, welche aus HSV gB oder gD, HIV gp 120, CMV gB, HCV E2, INFV HA oder NA und H. influenzae P6 besteht, ausgewählt ist.

8. Immunogene Liposomzusammensetzung nach Anspruch 1, wobei das antigene Konstrukt darüber hinaus eine Linkerregion enthält, welche die eine oder mehreren antigene(n) Determinante(n) mit der hydrophoben Domäne verbindet.

9. Immunogene Liposomzusammensetzung nach Anspruch 8, wobei die Linkerregion ein Peptid ist.

10. Immunogene Liposomzusammensetzung nach Anspruch 9, wobei die Linkerregion aus 1 bis etwa 200 Aminosäureresten besteht.

11. Immunogene Liposomzusammensetzung nach Anspruch 10, wobei die Aminosäurereste aus der Gruppe, welche aus Ala, Arg, Asn, Asp Cys, Glu, Gln, Gly, His, Ile, Leu, Lys, Met, Phe, Pro, Ser, Thr, Trp, Tyr und Val besteht, ausgewählt werden.

12. Immunogene Liposomzusammensetzung nach Anspruch 1, welche ein antigenes Konstrukt, das eine oder mehrere Antigendeterminanten und eine hydrophobe Domäne enthält, und einen pharmazeutisch annehmbaren Träger enthält.

13. Immunogene Liposomzusammensetzung nach Anspruch 12, wobei das antigene Konstrukt darüber hinaus eine Linkerregion enthält, welche die eine oder mehreren antigene(n) Determinante(n) mit der hydrophoben Domäne verbindet.

14. Immunogene Liposomzusammensetzung nach Anspruch 12, welche darüber hinaus ein Adjuvans oder mehrere Adjuvantien enthält.

15. Immunogene Liposomzusammensetzung nach Anspruch 1, wobei die eine oder mehreren antigenen Determinanten eine antigene Determinante eines bakteriellen Pathogens oder Toxins, eines viralen Pathogens, eines Pilzpathogens oder eines Parasitenpathogens ist.

16. Immunogene Liposomzusammensetzung nach einem der vorangehenden Ansprüche zur Verwendung in einem Verfahren zur Immunisierung eines Wirtstieres.

17. Immunogene Liposomzusammensetzung nach einem der vorangehenden Ansprüche zur Verwendung in einem Verfahren zur Auslösung einer Immunantwort in einem Wirtstier.

18. Immunogene Liposomzusammensetzung nach Anspruch 16 oder 17, wobei das Wirtstier ein Säugetier ist.

19. Immunogene Liposomzusammensetzung nach Anspruch 18, wobei das Säugetier ein Mensch ist.

20. Immunogene Liposomzusammensetzung nach Anspruch 19, wobei die antigene Determinante aus der Gruppe, welche aus HSV gB oder gD, HIV gp 120, CMV gB, HCV E2, INFV HA oder NA und H. influenzae P6 besteht, ausgewählt ist.

21. Immunogene Liposomzusammensetzung nach Anspruch 16 oder 17, wobei das Tier ein Vogel ist.

22. Verfahren zur Herstellung einer immunogenen Liposomzusammensetzung, welches die Schritte umfasst, dass
ein Gen exprimiert wird, welches ein wasserlösliches Fusionsprodukt kodiert, das eine Antigendeterminante und eine hydrophobe Domäne enthält; und dass
a) vesikelformende Lipide und das Fusionsprotein in einem geeigneten organischen Lösungsmittel gelöst werden;
durch das Verdunsten des organischen Lösungsmittels ein Lipidfilm oder ein sprühgetrocknetes Pulver gebildet wird;
der Lipidfilm oder das Pulver hydratisiert wird; und
der hydratisierte Lipidfilm oder das hydratisierte Pulver um eine immunogene Liposomzusammensetzung zu bilden, dispergiert wird; oder dass
b) das Fusionsprotein in einem wässrigen Puffer gelöst wird;
entweder ein Lipidpulver oder ein Lipidfilm mit dem Puffer, der das Fusionsprotein enthält, hydratisiert wird; und
das Lipidpulver oder der Lipidfilm, um eine immunogene Liposomzusammensetzung zu bilden, dispergiert wird; oder dass
c) das Fusionsprotein in einem wässrigen Puffer gelöst wird; und das Fusionsprotein zu bereits vorher gebildeten Liposomen hinzugefügt wird um eine immunogene Liposomzusammensetzung zu bilden.

23. Immunogene Liposomzusammensetzung, welche durch das Verfahren wie in Anspruch 22 beansprucht erhältlich ist.

## Revendications

1. Composition liposomique immunogénique comprenant :
des lipides formant une vésicule et
une construction protéique antigénique qui est un produit de fusion soluble aqueux comprenant un ou plusieurs déterminants antigéniques et un domaine hydrophobe, le domaine hydrophobe étant associé à la membrane de la composition liposomique immunogénique.

2. Composition liposomique immunogénique selon la revendication 1, comprenant en outre un ou plusieurs adjuvants.

3. Composition liposomique immunogénique selon la revendication 1, dans laquelle la construction antigénique est synthétisée chimiquement.

4. Composition liposomique immunogénique selon la revendication 1, dans laquelle le domaine hydrophobe est un peptide.

5. Composition liposomique immunogénique selon la revendication 4, dans laquelle le domaine hydrophobe comprend environ de 15 à environ 500 résidus d'acides aminés.

6. Composition liposomique immunogénique selon la revendication 5, dans laquelle le domaine hydrophobe comprend un ou plusieurs acides aminés choisis dans le groupe comprenant Ala, Asn, Cys, Gln, Gly, His, Ile, Leu, Met, Phe, Pro, Ser, Thr, Trp, Tyr et Val.

7. Composition liposomique immunogénique selon la revendication 1, dans laquelle le déterminant antigénique est dérivé d'un antigène choisi dans le groupe comprenant gB ou gD de HSV, gp 120 de HIV, gB de CMV, E2 de HCV, HA ou NA de INFV et P6 de H. influenzae.

8. Composition liposomique immunogénique selon la revendication 1, dans laquelle la construction antigénique comprend en outre une région de liaison reliant un ou plusieurs déterminants antigéniques au domaine hydrophobe.

9. Composition liposomique immunogénique selon la revendication 8, dans laquelle la région de liaison est un peptide.

10. Composition liposomique immunogénique selon la revendication 9, dans laquelle la région de liaison comprend 1 à environ 200 résidus d'acides aminés.

11. Composition liposomique immunogénique selon la revendication 10, dans laquelle les résidus d'acides aminés sont choisis dans le groupe comprenant Ala, Arg, Asn, Asp, Cys, Glu, Gln, Gly, His, Ile, Leu, Lys, Met, Phe, Pro, Ser, Thr, Trp, Tyr et Val.

12. Composition liposomique immunogénique selon la revendication 1, comprenant une construction antigénique comprenant un ou plusieurs déterminants antigéniques et un domaine hydrophobe et un véhicule pharmaceutiquement acceptable.

13. Composition liposomique immunogénique selon la revendication 2, dans laquelle la construction antigénique comprend en outre une région de liaison reliant un ou plusieurs déterminants antigéniques au domaine hydrophobe.

14. Composition liposomique immunogénique selon la revendication 2, comprenant en outre un ou plusieurs adjuvants.

15. Composition liposomique immunogénique selon la revendication 1, dans laquelle un ou plusieurs déterminants antigéniques est/sont un déterminant antigénique d'un agent pathogène ou d'une toxine bactérien(ne), d'un agent pathogène viral, d'un agent pathogène fongique ou d'un agent pathogène parasite.

16. Composition liposomique immunogénique selon l'une quelconque des revendications précédentes à utiliser dans un procédé d'immunisation d'un animal hôte.

17. Composition liposomique immunogénique selon l'une quelconque des revendications précédentes, à utiliser dans un procédé d'induction d'une réponse immunogénique chez un animal hôte.

18. Composition liposomique immunogénique selon la revendication 16 ou 17, dans laquelle l'animal hôte est un mammifère.

19. Composition liposomique immunogénique selon la revendication 18, dans laquelle le mammifère est un être humain.

20. Composition liposomique immunogénique selon la revendication 19, dans laquelle le déterminant antigénique est choisi dans le groupe comprenant gB ou gD de HSV, gp120 de HIV, gB de CMV, E2 de HCV, HA ou NA de INFV et P6 de H. influenzae.

21. Composition liposomique immunogénique selon la revendication 16 ou 17, dans laquelle l'animal est un oiseau.

22. Procédé de production d'une composition liposomique immunogène comprenant :
l'expression d'un gène qui code pour une protéine de fusion soluble aqueuse comprenant un déterminant antigénique et un domaine hydrophobe et
(a) la dissolution de lipides formant une vésicule et de la protéine de fusion dans un solvant organique approprié
la formation d'un film lipidique ou d'une poudre séchée par pulvérisation par évaporation du solvant organique
l'hydratation du film lipidique ou de la poudre et
la dispersion du film lipidique hydraté ou de la poudre pour former une composition liposomique immunogénique ou
(b) la dissolution de la protéine de fusion dans un tampon aqueux
l'hydratation d'une poudre lipidique ou d'un film lipidique avec le tampon contenant la protéine de fusion et
la dispersion de la poudre lipidique ou du film pour former une composition liposomique immunogénique ; ou
(c) la dissolution de la protéine de fusion dans un tampon aqueux et
l'addition de la protéine de fusion à des liposomes préformés pour former une composition liposomique immunogénique.

23. Composition liposomique immunogénique pouvant être obtenue par un procédé selon la revendication 22.
